# EUROPEAN PATENT SPECIFICATION

(11) **EP 4 491 167 B1**
(45) Date of publication and mention of the grant of the patent: **30.09.2026**
(21) Application number: 23819782.6
(22) Date of filing: 02.06.2023
(51) Int. Cl.: A61F 13/49, A61F 13/496, A61F 13/51

(54) **STRETCHABLE SHEET, ABSORBENT ARTICLE, AND UNDERPANTS-STYLE ABSORBENT ARTICLE**
DEHNBARE FOLIE, SAUGFÄHIGER ARTIKEL UND SAUGFÄHIGER ARTIKEL VOM UNTERHOSEN-TYP
FEUILLE ÉTIRABLE, ARTICLE ABSORBANT ET ARTICLE ABSORBANT DE TYPE CULOTTE

(30) Priority: 10.06.2022 JP 2022094703
(43) Date of publication of application: 15.01.2025
(73) Proprietor: Unicharm Corporation, Ehime 799-0111 (JP)
(72) Inventor: Anno, Mika, Kanonji-shi, Kagawa 769-1602 (JP); Noma, Shinji, Kanonji-shi, Kagawa 769-1602 (JP); NAGAI, Takahito, Kanonji-shi, Kagawa 769-1602 (JP); Nara, Yuki, Kanonji-shi, Kagawa 769-1602 (JP); YOKOYAMA, Takumi, Kanonji-shi, Kagawa 769-1602 (JP)
(74) Representative: Dolleymores
(86) International application number: PCT/JP2023/020677
(87) International publication number: WO 2023/238802

(56) References cited:
- BR-A2- 112021 016 164
- JP-A- 2016 043 247
- JP-A- 2020 179 047
- JP-A- 2022 062 384
- US-A1- 2019 070 042
- US-A1- 2021 282 979
- US-B2- 10 980 678
- US-B2- 10 987 255

## Description

### [Technical Field]

The present invention relates a stretchy sheet, an absorbent article, and an underpants-shaped absorbent article.

### [Background Art]

Patent Document 1 discloses a stretchy sheet in which an elastic member (wire-like element) is arranged in folds of a sheet (tubular sleeve), and connection portions for connecting two sheets are formed on two sides in a transverse direction of the elastic member. In the state where the tension of the elastic member is exerted, the elastic member is removable from between the connection portions, meanwhile, in the state where the tension thereof is not exerted, the elastic member is secured at narrow portions between the connection portions. Patent Document 2 discloses a stretchable/contractible sheet having an up-down direction and a left-right direction intersecting each other and includes a first sheet; a second sheet; welded portions; and elastic members.

### [Citation List]

### [Patent Literature]

[Patent Document 1] Japanese Patent No.3212615
[Patent Document 2] US Patent No. 10987255

### [Summary of Invention]

### [Technical Problem]

In the connection portions (welded portions) formed using heat sealing or the like, the sheet has a high stiffness. Accordingly, by reducing the length of the welded portions (length along the elastic member) that sandwich the elastic member, the flexibility of the sheet can be ensured. However, reduction in the length of welded portions reduces the force to sandwich and secure the elastic member at the narrow part between welded portions (between a welded portion pair), thereby being unable to secure the elastic member that is contracting, and thus the elastic member becomes more likely to come out from between the welded portion pair. This reduces the stretchability of the sheet.

The present invention is directed to suppression of a case where an elastic member comes out of a welded portion pair while ensuring the flexibility of stretchy sheet, thereby suppressing reduction in the stretchability of the stretchy sheet.

### [Solution to Problem]

A main aspect of the invention is a stretchy sheet having a vertical direction and a lateral direction intersecting each other, the stretchy sheet comprising: a pair of sheets; a plurality of welded portions joining the pair of sheets; and a plurality of elastic members capable of stretching and contracting in the lateral direction, the plurality of elastic members being arranged with a space in the vertical direction and between the pair of sheets that are joined by the plurality of welded portions, the plurality of welded portions including a plurality of welded portion pairs, the plurality of welded portion pairs including a welded portion pair located on two sides of an elastic member in the vertical direction, the elastic member being included in the plurality of elastic members, the elastic member, in a state of contracting in the lateral direction, being sandwiched between the welded portion pair, thereby restricting a position of the elastic member in the lateral direction with respect to the pair of sheets, the plurality of welded portion pairs including a first welded portion pair and a second welded portion pair that is located adjacent to the first welded portion pair on one side in the lateral direction, both the first welded portion pair and the second welded portion pair sandwiching a first elastic member, the first elastic member being included in the plurality of elastic members, the first welded portion pair including a first welded portion and a second welded portion that are located with a first space therebetween in the lateral direction, on a same side in the vertical direction with respect to the first elastic member, in a state where the stretchy sheet is stretched in the lateral direction, a first length being a lateral length from a lateral end on the one side of the first welded portion pair to a lateral end on another side of the second welded portion pair, the first length being longer than a lateral length of the first space. Other features of the present disclosure will become apparent from the description in the present specification and the accompanying drawings.

### [Advantageous Effects of Invention]

According to the present invention, it is possible to suppress a case where an elastic member comes out from between a welded portion pair while ensuring the flexibility of stretchy sheet, thereby suppressing reduction in the stretchability of the stretchy sheet.

### [Brief Description of Drawings]

FIG. 1 is a schematic perspective view of a pull-on disposable diaper 1.
FIG. 2 is a schematic plan view of a diaper 1 in an unfolded and stretched state, as viewed from the skin surface side of a wearer.
FIG. 3 a schematic cross-sectional view taken along a line A-A in FIG. 2.
FIG. 4 is an illustrative diagram of welded-portion rows Ra, Rb provided in a front waist portion 20.
FIGS. 5A and 5B are illustrative diagrams showing a function of attaching a waist elastic member 23.
FIG. 6A is an enlarged view of portions of welded portion rows Ra, Rb, and FIG. 6B is an illustrative diagram of a normal welded portion pair 51b.
FIG. 7 is an illustrative diagram of a spaced welded portion pair 51a.
FIG. 8A is an illustrative diagram of spaced welded portion pair 51a in a stretched state, and FIGS. 8B and 8C are illustrative diagrams of spaced welded portion pair 51a in a contracted state.
FIGS. 9A to 9C are illustrative diagrams of variations of spaced welded portion pairs 51a.
FIGS. 10A and 10B are illustrative diagrams of variations of spaced welded portion pairs 51a.
FIG. 11 is a diagram showing a relationship between non-welded portions 52 and a pattern of compressed portions 60, 61 in a skin-side sheet 21 and a non-skin-side sheet 22.
FIGS. 12A and 12B are illustrative diagrams of a waist elastic member 23.
FIG. 13 is an illustrative diagram of spaced welded portion pairs 51a provided at a side portion of an absorbent main body 10 and in the vicinity of a side joining portion SS.
FIG. 14 is a plan view of a tape-type diaper 2 in a state of being unfolded in a longitudinal direction.

### [Description of Embodiments]

At least following matters will become apparent from the descriptions of the present specification and the accompanying drawings.

Aspect 1 is a stretchy sheet having a vertical direction and a lateral direction intersecting each other, the stretchy sheet comprising: a pair of sheets; a plurality of welded portions joining the pair of sheets; and a plurality of elastic members capable of stretching and contracting in the lateral direction, the plurality of elastic members being arranged with a space in the vertical direction and between the pair of sheets that are joined by the plurality of welded portions, the plurality of welded portions including a plurality of welded portion pairs, the plurality of welded portion pairs including a welded portion pair located on two sides of an elastic member in the vertical direction, the elastic member being included in the plurality of elastic members, the elastic member, in a state of contracting in the lateral direction, being sandwiched between the welded portion pair, thereby restricting a position of the elastic member in the lateral direction with respect to the pair of sheets, the plurality of welded portion pairs including a first welded portion pair and a second welded portion pair that is located adjacent to the first welded portion pair on one side in the lateral direction, both the first welded portion pair and the second welded portion pair sandwiching a first elastic member, the first elastic member being included in the plurality of elastic members, the first welded portion pair including a first welded portion and a second welded portion that are located with a first space therebetween in the lateral direction, on a same side in the vertical direction with respect to the first elastic member, in a state where the stretchy sheet is stretched in the lateral direction, a first length being a lateral length from a lateral end on the one side of the first welded portion pair to a lateral end on another side of the second welded portion pair, the first length being longer than a lateral length of the first space.

According to Aspect 1, in a non-welded portion corresponding to the first space having a length smaller than the first length, which is an interval between the welded portion pairs, the sheet contracts so that the fiber density increases. This causes fibers to be more likely to be entangled with the elastic member in the non-welded portion, thereby being able to suppress the case where the elastic member comes out from between the first welded portion pair, which ensures the stretchability of the stretchy sheet. Further, the first welded portion pair has the non-welded portion, and the space between the welded portion pair adjacent in the lateral direction is wide, so that the welded portions are not closely arranged in the lateral direction. This ensures the flexibility of the stretchy sheet as well.

Aspect 2 is the stretchy sheet according to Aspect 1, wherein in the state where the stretchy sheet is stretched in the lateral direction, the lateral length of the first space is equal to or less than a lateral length of the first welded portion, and equal to or less than a lateral length of the second welded portion.

According to Aspect 2, it is possible to firmly sandwich the sheet (first space) between the first welded portion and second welded portion that have long lengths, thereby being able to increase the fiber density of the sheet. This makes it easier for the fibers to be entangled with the elastic member, and can suppress the case where the elastic member comes out of the first welded portion pair.

Aspect 3 is the stretchy sheet according to Aspect 1 or 2, wherein in the state where the stretchy sheet is stretched in the lateral direction, the first length is longer than a lateral length of the first welded portion pair.

According to Aspect 3, in the first welded portion pair, a plurality of welded portions are adjacent with the first space therebetween, which results in the lateral length thereof being relatively long, and the welded portion pairs are arranged in the lateral direction at an interval (first length) larger than the length of the first welded portion pair, thereby ensuring the flexibility of the stretchy sheet.

Aspect 4 is the stretchy sheet according to any of Aspects 1 to 3, wherein the second welded portion pair including a third welded portion and a fourth welded portion that are located with a second space therebetween in the lateral direction, on the same side in the vertical direction with respect to the first elastic member, and in the state where the stretchy sheet is stretched in the lateral direction, the first length is longer than a lateral length of the second space.

According to Aspect 4, the fibers are more likely to be entangled with the elastic member in the non-welded portion corresponding to the second space, not only in the first welded portion pair but also in the second welded portion pair. This can more reliably suppress the case where the elastic member come out of the welded portion pair, thereby ensuring the stretchability of the stretchy sheet.

Aspect 5 is the stretchy sheet according to any of Aspects 1 to 4, wherein the first welded portion pair includes, on the same side in the vertical direction with respect to the first elastic member, the first welded portion and the second welded portion, and one or more fifth welded portions arranged to be spaced apart therefrom in the lateral direction, and in the state where the stretchy sheet is stretched in the lateral direction, the first length is longer than a lateral length of a space between the one fifth welded portion and the first welded portion or another fifth welded portion, the first welded portion and the other fifth welded portion being adjacent to the one fifth welded portion in the lateral direction, the other fifth welded portion being included in the fifth welded portions.

According to Aspect 5, it is possible to increase the fiber density of the sheet in the non-welded portion corresponding to a space having a length smaller than the first length, which is an interval between the welded portion pairs, and further, with the presence of two or more non-welded portions having an increased fiber density, it is possible to further suppress the case where the elastic member comes out of the first welded portion pair.

Aspect 6 is the stretchy sheet according to Aspect 5, wherein in the state where the stretchy sheet is stretched in the lateral direction, the lateral length of the space between the one fifth welded portion and the first welded portion or the other fifth welded portion adjacent to the one fifth welded portion in the lateral direction is equal to or less than each of lateral lengths of the first welded portion, the second welded portion, and the fifth welded portion.

According to Aspect 6, the first welded portions, second welded portions, and fifth welded portion(s), which have long lengths, can firmly sandwich the sheet therebetween, thereby being able to increase the fiber density of the sheet. This makes it easier for the fibers to be entangled with the elastic member, thereby being able to suppress the case where the elastic member comes out of the first welded portion pair.

Aspect 7 is the stretchy sheet according to any of Aspects 1 to 6, wherein the first welded portion pair includes a sixth welded portion and a seventh welded portion that are located with a third space therebetween in the lateral direction, on a side opposite to the same side in the vertical direction with respect to the first elastic member, and in the state where the stretchy sheet is stretched in the lateral direction, the first length is longer than a lateral length of the third space.

According to Aspect 7, with the non-welded portions being located on two sides in the vertical direction of the elastic member, the non-welded portions are more likely to contract along the lateral direction, so that the fiber density is more likely to increase. In addition, the fibers are more likely to be entangled with the elastic member from both sides in the vertical direction, thereby being able to further suppress the case where the elastic member comes out of the first welded portion pair.

Aspect 8 is the stretchy sheet according to Aspect 7, wherein in the state where the stretchy sheet is stretched in the lateral direction, the first space and the third space have a region in which the first space and the third space overlap in the lateral direction, and a lateral center of the first space is shifted in the lateral direction from a lateral center of the third space.

According to Aspect 8, in a portion in which the non-welded portion corresponding to the first space or third space overlaps with the welded portion in the lateral direction, the elastic member is subjected to a force from the welded portion on one side in the vertical direction while being subjected to a force from the non-welded portion with higher fiber density on the other side in the vertical direction. Thus, the fibers are more likely to be entangled with the elastic member.

Aspect 9 is the stretchy sheet according to Aspect 8, further including: a plurality of eighth welded portions arranged to be spaced apart from the first welded portion in the vertical direction, and a plurality of ninth welded portions arranged to be spaced apart from the second welded portion in the vertical direction, wherein an eighth welded portion of the eighth welded portions is shifted in the lateral direction from the first welded portion or another eighth welded portion of the eighth welded portions, the first welded portion and the other eighth welded portion being adjacent to the eighth welded portion in the vertical direction, a ninth welded portion of the ninth welded portions is shifted in the lateral direction from the second welded portion or another ninth welded portion of the ninth welded portions, the second welded portion and the other ninth welded portion being adjacent to the ninth welded portion in the vertical direction, and in the state where the stretchy sheet is stretched in the lateral direction, the first length is longer than a lateral length of a space between the eighth welded portion and the ninth welded portion, the eighth welded portion and the ninth welded portion being adjacent in the lateral direction.

According to Aspect 9, a portion of the elastic member located in the non-welded portion that corresponds to the first space overlaps, in the lateral direction, with the eighth welded portions and ninth welded portions arranged in the vertical direction. Thus, the elastic member is more likely to be affected by the eighth and ninth welded portions, which causes the fibers to be more likely to be entangled with the elastic member.

Aspect 10 is the stretchy sheet according to any of Aspects 1 to 9, wherein each of the pair of sheets has a plurality of compressed portions in which the each of the pair of sheets is compressed in a thickness direction thereof, and when the stretchy sheet in a laterally stretched state is viewed from above, at least a part of the plurality of compressed portions included in one of the pair of sheets has a region that is not overlapped with the compressed portions included in another sheet of the pair of sheets.

According to Aspect 10, with the compressed portions in the pair of sheets not completely overlapped with each other, non-compressed fibers in one of the pair of sheets are more likely to be located in the non-welded portion corresponding to the first space. This makes it easier for the fibers to be entangled with the elastic member in the non-welded portion, thereby being able to suppress the case where the elastic member comes out of the first welded portion pair.

Aspect 11 is the stretchy sheet according to any of Aspects 1 to 10, wherein at least one of the pair of sheets includes a plurality of compressed portions in which the at least one of the pair of sheets is compressed in a thickness direction thereof, and when the stretchy sheet in a laterally stretched state is viewed from above, the first space has a region that is not overlapped with the compressed portions.

According to Aspect 11, the non-compressed fibers of the sheet are located in the non-welded portion corresponding to the first space, which makes it easier for the fibers to be entangled with the elastic member in the non-welded portion, thereby being able to suppress the case where the elastic member comes out of the first welded portion pair.

Aspect 12 is the stretchy sheet according to any of Aspects 1 to 11, wherein at least one of the pair of sheets has a plurality of compressed portions in which the at least one of the pair of sheets is compressed in a thickness direction thereof, and in the state where the stretchy sheet is stretched in the lateral direction, the lateral length of the first space is equal to or less than a maximum value of a lateral interval between the compressed portions adjacent in the lateral direction.

According to Aspect 12, the non-welded portion corresponding to the first space is more likely to be located between the compressed portions adjacent in the lateral direction. This makes it easier for the fibers to be entangled with the elastic member in the non-welded portion, thereby being able to suppress the case where the elastic member comes out of the first welded portion pair.

Aspect 13 is the stretchy sheet according to any of Aspects 1 to 12, wherein when twice a length of a thickness of at least one of the pair of sheets is defined as a second length, and in the state where the stretchy sheet is stretched in the lateral direction, the lateral length of the first space is equal to or more than the second length.

According to Aspect 13, the non-welded portion corresponding to the first space is not too narrow, allowing the sheet to be bent in the non-welded portion, thereby being able to increase the fiber density of the sheet. Thus, it is possible to suppress the case where the elastic member comes out of the first welded portion pair.

Aspect 14 is the stretchy sheet according to any of Aspects 1 to 13, wherein the first elastic member in a twisted state is arranged between the pair sheets.

According to Aspect 14, the dense fibers in the non-welded portion corresponding to the first space are more likely to be entangled with the twisted portion of the elastic member, thereby being able to suppress the case where the elastic member comes out of the welded portion pair.

Aspect 15 is the stretchy sheet according to any of Aspects 1 to 14, wherein the first elastic member is a string-like elastic member in which a plurality of elastic fibers are assembled, and has a first outer diameter portion having an outer diameter that is a first outer diameter, and a second outer diameter portion having an outer diameter smaller than the first outer diameter.

According to Aspect 15, even if a part of the elastic fibers break, connection can be provided with the remaining elastic fibers, thereby being able to suppress the case where the elastic member breaks. In addition, a strong frictional force is more likely to be generated between the welded portions in the first outer diameter portion having a large diameter, which makes it easier for the fibers in the non-welded portion corresponding to the first space to be entangled. This can further suppress the case where the elastic member comes out of the welded portion pair.

Aspect 16 is the stretchy sheet according to any of Aspects 1 to 15, wherein the plurality of welded portion pairs include a third welded portion pair that is located adjacent to the first welded portion pair on the other side in the lateral direction, the third welded portion pair sandwiching the first elastic member, and in the state where the stretchy sheet is stretched in the lateral direction, a lateral length from a lateral end on the other side of the first welded portion pair to a lateral end on the one side of the third welded portion pair is longer than the lateral length of the first space.

According to Aspect 16, the welded portion pairs are located on two sides in the lateral direction of the first welded portion pair with a relatively wide interval (first length) therebetween. This can suppress the case where the welded portion pairs are closely arranged in the lateral direction, thus ensuring the flexibility of the stretchy sheet.

Aspect 17 is an absorbent article including the stretchy sheet according to any of Aspects 1 to 16, the absorbent article having a vertical direction and a lateral direction intersecting each other, wherein the vertical direction of the stretchy sheet is aligned with the vertical direction of the absorbent article, the lateral direction of the stretchy sheet is aligned with the lateral direction of the absorbent article, the first elastic member includes a non-continuous portion in which the first elastic member is cut to be non-continuous in a lateral central portion of the absorbent article, and continuous portions located on outer sides in the lateral direction with respect to the non-continuous portion, and the first welded portion pair sandwiches an end portion on a central side in the lateral direction of each of the continuous portions.

According to Aspect 17, the fibers of the sheet in the non-welded portion corresponding to the first space are more likely to be entangled with a cut-end portion of the elastic member that has been cut at the non-continuous portion, and thus the elastic member is less likely to come out of the first welded portion pair, thereby ensuring the stretchability of the stretchy sheet.

Aspect 18 is an absorbent article including the stretchy sheet according to any of Aspects 1 to 17, the absorbent article having a vertical direction and a lateral direction intersecting each other, wherein the vertical direction of the stretchy sheet is aligned with the vertical direction of the absorbent article, the lateral direction of the stretchy sheet is aligned with the lateral direction of the absorbent article, the stretchy sheet has a fourth welded portion pair that sandwiches the first elastic member, the fourth welded portion pair has the plurality of welded portions arranged with a space in the lateral direction, on the same side in the vertical direction with respect to the first elastic member, the first welded portion pair is located in each lateral end portion of the absorbent article, the fourth welded portion pair is located in a lateral central portion of the absorbent article, and a lateral length of the first welded portion pair is longer than a lateral length of the fourth welded portion pair.

According to Aspect 18, in the lateral ends of the absorbent article, the elastic member is more likely to break, since the side joining portions of the pants-typed diaper, the fastening tapes of the tape-type diaper or the like are located therein. Thus, the first welded portion pair having a long length can suppress the case where the elastic member comes out of the first welded portion pair, thereby ensuring the stretchability of the stretchy sheet. Meanwhile, with a reduction in the length of the fourth welded portion pair, it is possible to ensure the flexibility of the stretchy sheet, while suppressing the case where the elastic member comes out of the fourth welded portion pair.

Aspect 19 is an underpants-shaped absorbent article including the stretchy sheet according to Aspects 1 to 18, the underpants-shaped absorbent article having a vertical direction and a lateral direction intersecting each other, the underpants-shaped absorbent article, comprising: an absorbent main body: and a pair of waist portions, the stretchy sheet being provided to at least one of the pair of waist portions, the vertical direction of the stretchy sheet being aligned with the vertical direction of the underpants-shaped absorbent article, the lateral direction of the stretchy sheet being aligned with the lateral direction of the underpants-shaped absorbent article, the pair of waist portions being joined, at lateral side portions thereof, by a pair of side joining portions, and in the state where the underpants-shaped absorbent article is stretched in the lateral direction, at least a part of the first welded portion pair being located in a region that is located on an inner side in the lateral direction, by the first length, from a lateral inner end of each of the side joining portions.

According to Aspect 19, even if the elastic member breaks at the side joining portion, the elastic member is more likely to be firmly sandwiched by the first welded portion pair in the vicinity of the side joining portion, thereby ensuring the stretchability over a wide range in the lateral direction of the stretchy sheet.

### First Embodiment

A first embodiment will be described using an example of a pull-on disposable diaper for adults as an absorbent article (underpants-shaped absorbent article) using a stretchy sheet according to a present embodiment.

### Basic configuration of pull-on disposable diaper 1

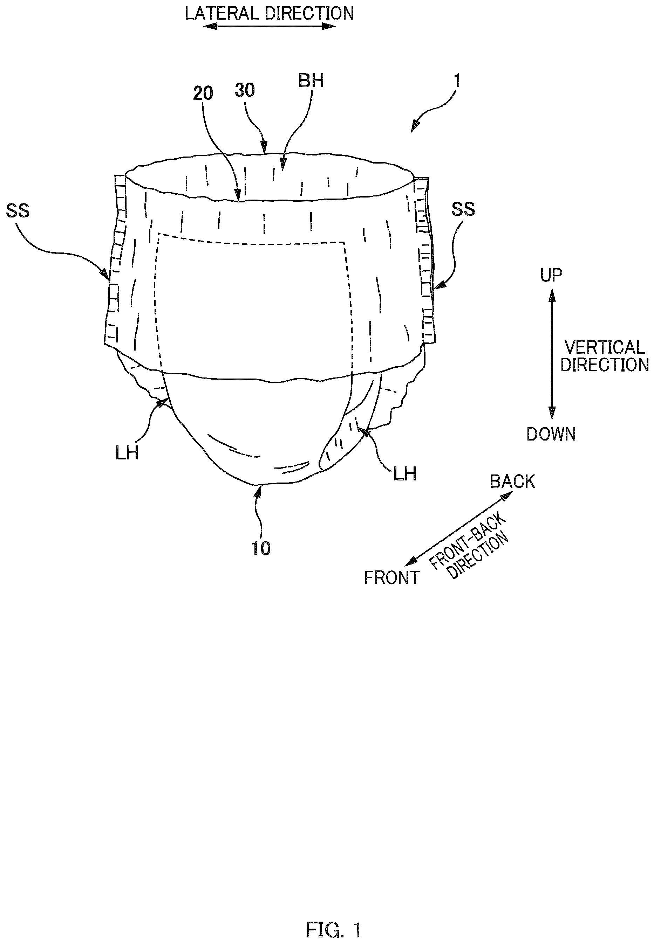

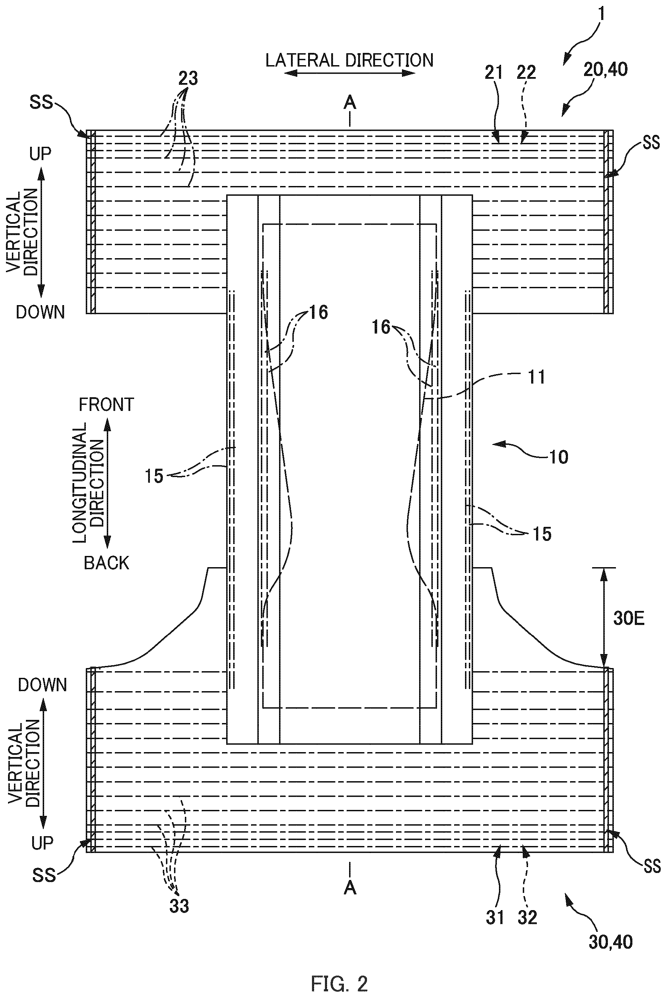

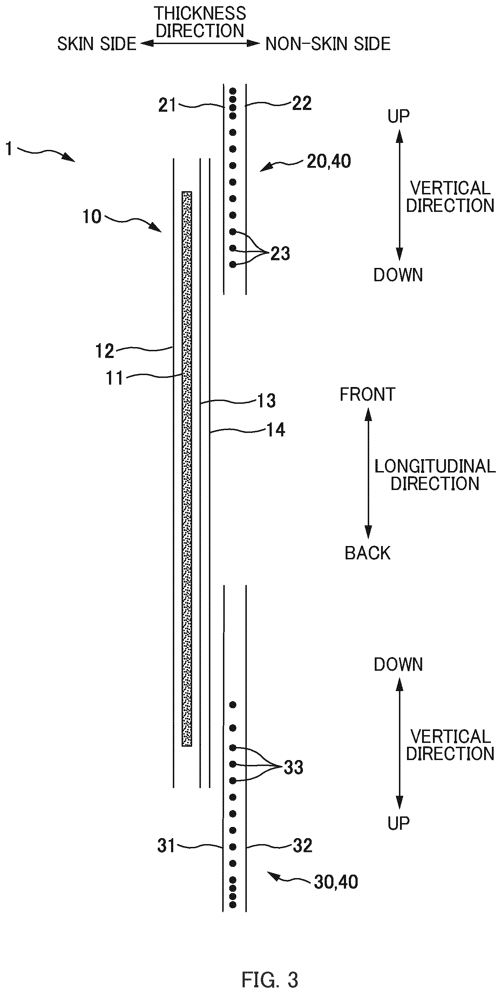

FIG. 1 is a schematic perspective view of a pull-on disposable diaper 1 (hereinafter, also referred to as "diaper"). FIG. 2 is a schematic plan view of a diaper 1 in an unfolded and stretched state, as viewed from the skin surface side of a wearer. FIG. 3 a schematic cross-sectional view taken along a line A-A in FIG. 2.

The diaper 1 has a vertical direction, a lateral direction, and a front-back direction that are perpendicular to one another, and has a waist opening BH and a pair of leg openings LH. In the vertical direction, the side corresponding to the waist opening BH is the upper side, and the side corresponding to the wearer's crotch is the lower side. In the front-back direction, the side corresponding to the wearer's stomach is the front side, and the side corresponding to the wearer's back is the back side. In addition, the diaper 1 has a thickness direction in which materials are layered as shown in FIG. 3, and in the thickness direction, the side that comes into contact with the wearer will be referred to as skin side, and the side opposite thereto will be referred to as non-skin side.

The diaper 1 includes an absorbent main body 10 and a pair of waist portions 20, 30. The pair of waist portions 20, 30 is joined to the non-skin-side surface of the absorbent main body 10 with an adhesive or the like. The one on the front side out of the pair of waist portions 20 is also referred to as front waist portion 20, and the one on the back side is also referred to as back waist portion 30. The front waist portion 20 and the back waist portion 30 are joined by a pair of side joining portions SS at lateral side portions. Examples of the joining method by the side joining portions SS include thermal welding, ultrasonic welding, and joining with an adhesive.

As shown in FIG. 3, the absorbent main body 10 has an absorbent core 11, a liquid-permeable top sheet 12 arranged on the skin side with respect to the absorbent core 11, a liquid-impermeable back sheet 13 arranged on the non-skin side with respect to the absorbent core 11, and an exterior sheet 14. The absorbent core 11 may be any material that absorbs and retains excreted fluid, and examples thereof include those formed by molding liquid absorbent material such as pulp fibers and superabsorbent polymer into a predetermined shape. The absorbent core 11 may be covered with a liquid permeable sheet such as tissue paper, nonwoven fabric, or the like.

As shown in FIG. 2, in the two lateral sides of the absorbent main body 10, leg elastic members 15 (e.g., elastic strings) may be provided, which stretches and contracts along a longitudinal direction of the absorbent main body 10. Furthermore, on the inner side in the lateral direction with respect to the leg elastic members 15, leak-proof wall portions capable of standing up toward the skin side may be provided. The leak-proof wall portions are formed as follows, for example, the two lateral side portions of the exterior sheet 14 are folded inward in the lateral direction so as to be located on the skin-side surface of the top sheet 12, and the leak-proof wall elastic members 16 (e.g., elastic strings) that stretch and contract in the longitudinal direction are provided in the folded portion.

In the present embodiment, portions overlapped with the side joining portions SS in the vertical direction correspond to the waist portions 20, 30, and the front waist portion 20 and the back waist portion 30 are portions that are rectangular in a plan view. The back waist portion 30 has a substantially trapezoidal shaped extension portion 30E on the crotch side thereof. The extension portion 30E can cover the wearer's buttocks portion. In the following description, the back waist portion 30 and the extension portion 30E are also collectively referred to as back exterior portions 30, 30E. However, there is no limitation to the above configuration and, for example, it may have a configuration without the extension portion 30E on the back side, or may have a configuration including a portion extending from the front waist portion 20 toward the crotch side.

The front waist portion 20 and the back exterior portions 30, 30E each have skin-side sheets 21, 31, non-skin-side sheets 22, 32, and waist elastic members 23, 33 (e.g., elastic strings) that can stretch and contract in the lateral direction. The waist elastic members 23, 33 are arranged side by side with a space in the vertical direction, between the skin-side sheet 21, 31 and the non-skin-side sheet 22, 32.

It is preferable that the skin-side sheets 21, 31 and the non-skin-side sheets 22, 32 are each a flexible sheet member, and examples thereof includes spunbond nonwoven fabric, meltblown nonwoven fabric, SMS (spunbond/meltblown/spunbond) nonwoven fabric, and the like. The constituent fibers of the nonwoven fabric are not limited to mono fibers made of polypropylene (PP), which is a typical example of thermoplastic resin, but mono fibers made of another thermoplastic resin such as polyethylene (PE) may be used, and further composite fibers having a core-sheath structure including PE, PP, and the like may be used.

The basic configuration of the diaper 1 has been described above, but the above configuration of the diaper 1 is merely an example and is not intended to be limited thereto. For example, in the diaper 1 of the present embodiment, the front waist portion 20 and the back exterior portions 30, 30E are constituted by separate members, and the non-skin-side surface of the absorbent main body 10 is exposed in the crotch portion. There is no limitation thereto and, for example, the diaper may have an exterior member constituted by three components: the front waist portion 20, the back exterior portions 30, 30E, and the crotch portion connecting them, or the diaper may have an exterior member constituted by a single component that extends continuously from the front waist portion 20 to the back waist portion 30.

### Waist portions 20, 30 (stretchy sheet 40)

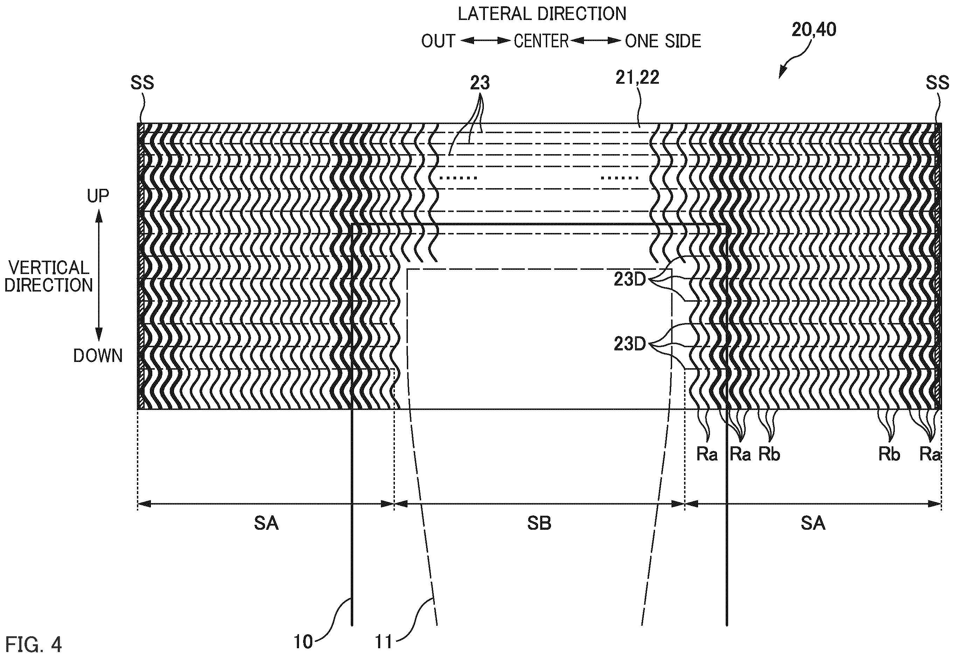

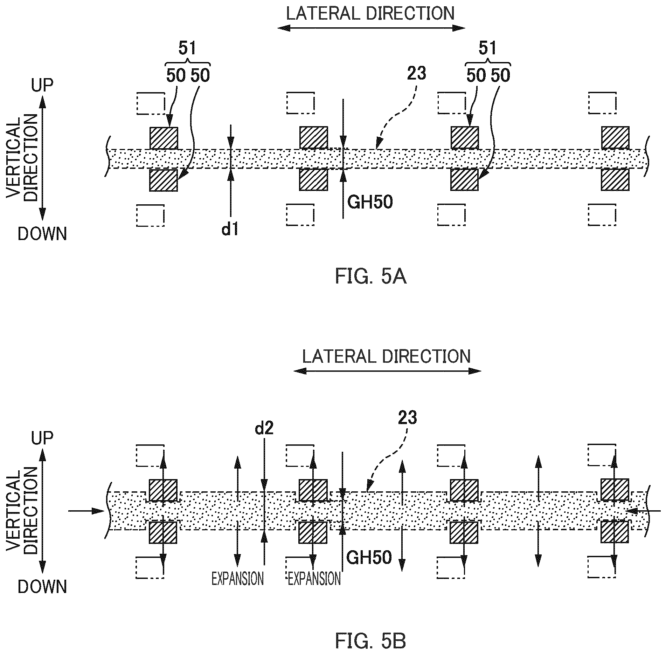

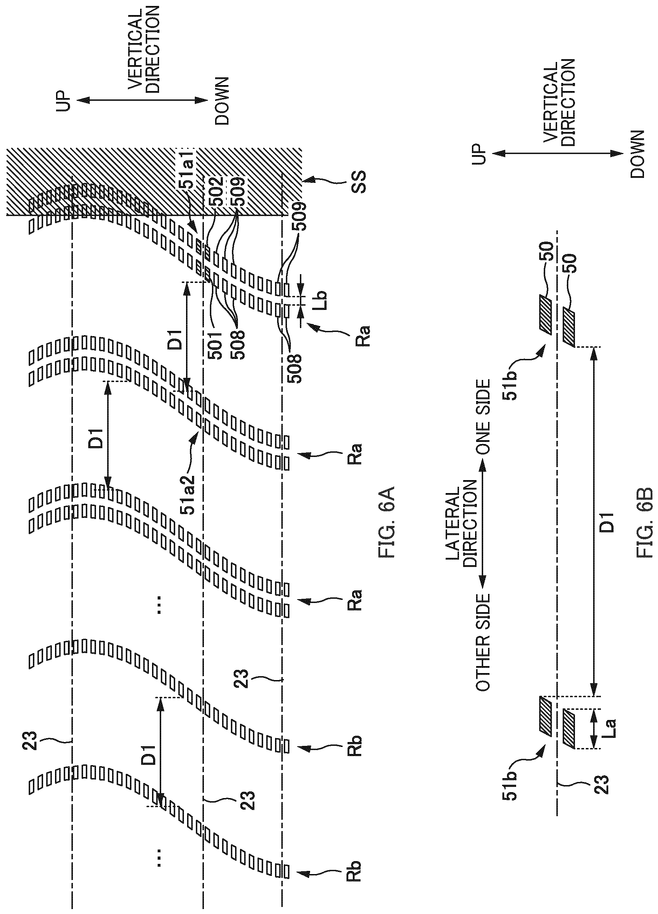

FIG. 4 is an illustrative diagram of welded-portion rows Ra, Rb provided in the front waist portion 20. FIGS. 5A and 5B are illustrative diagrams showing a function of attaching the waist elastic member 23. FIGS. 6A is an enlarged view of portions of the welded portion rows Ra, Rb, and FIG. 6B is an illustrative diagram of a normal welded portion pair 51b.

FIGS. 4, 6A, 6B, and the like show a state where the front waist portion 20 (the stretchy sheet 40) stretches in the lateral direction. The state where the waist portions 20, 30 and/or the stretchy sheet 40 stretches in the lateral direction refers to the state where they are stretched to the extent that creases formed in the waist portions 20, 30 and/or the stretchy sheet 40 can substantially no longer be seen, and the dimensions of the members constituting the waist portions 20, 30 and/or the stretchy sheet 40 (e.g., the skin-side sheet 21, etc.) match or are close to the dimensions of the members of their own (i.e., the dimensions when stretchability of the elastic members is not exhibited) .

### Position restriction of waist elastic members 23

The front waist portion 20 and the back waist portion 30 are constituted by the stretchy sheet 40 in which the waist elastic members 23, 33 (elastic members) that can stretch and contract in the lateral direction are arranged side-by-side with a space in the vertical direction, between the skin-side sheets 21, 31 and the non-skin-side sheets 22, 32 (between sheets in a pair) that are joined with the welded portions 50. As shown in FIG. 6, the skin-side sheets 21, 31 and the non-skin-side sheets 22, 32 are joined to each other by the welded portions 50 that are arranged discretely in the vertical direction and the lateral direction. Examples of the method of forming the welded portions 50 can include a known method such as ultrasonic welding and heat welding.

A pair of sheets that are joined by the welded portions 50 may be two separate sheets such as the skin-side sheets 21, 31 and the non-skin-side sheets 22, 32 in the present embodiment, or may be a sheet having two layers obtained by folding back a single sheet. Further, the welded portions 50 are not limited to joining only the pair of sheets sandwiching the waist elastic members 23, 33, but may join sheets with three or more layers including sheet overlaid on the pair of sheets.

Since the front waist portion 20 and the back waist portion 30 have substantially the same configuration, the following describes the front waist portion 20 as a representative of both. The front waist portion 20 (the stretchy sheet 40) has the multiple welded portion rows Ra, Rb in which the multiple welded portions 50 are arranged in the vertical direction. The multiple welded-portion rows Ra, Rb are arranged with a space in the lateral direction. As shown in FIG. 4, the welded-portion rows Ra, Rb in the present embodiment extend in the vertical direction while meandering in the lateral direction. In other words, as shown in FIG. 6, the positions of the welded portions 50 adjacent in the vertical direction are shifted in the lateral direction.

Meanwhile, the positions in the vertical direction of the welded portions 50 adjacent in the lateral direction are aligned. Thus, as shown in FIG. 5A, the positions of the spaces through which the waist elastic members 23 extend between the welded portions 50 adjacent to each other in the vertical direction are aligned in the vertical direction, so that the waist elastic members 23 can be arranged along the lateral direction. However, there is no limitation to this configuration, and the positions of the welded portions 50 adjacent to each other in the lateral direction may be shifted in the vertical direction, or the waist elastic members 23 may extend between the welded portions 50 whose positions are different in the vertical direction. In these cases as well, the front waist portion 20 can be given stretchability in the lateral direction.

As shown in FIG. 5A, two welded portions 50 located on two sides of the waist elastic member 23 in the vertical direction are also referred to as "welded portion pair 51". An upper welded portion 50 and a lower welded portion 50 that form the welded portion pair 51 are aligned in the vertical direction with a space GH50 therebetween. The size of the space GH50 is set to be equal to or slightly larger than an outer diameter D1 of the waist elastic member 23 when the front waist portion 20 is stretched to the maximum lateral length (GH50 ≥ d1).

However, the waist elastic member 23 becomes thicker as it contracts in the lateral direction. Thus, as shown in FIG. 5B, the space GH50 of the welded portion pair 51 is made smaller than the maximum outer shape D2 of the waist elastic member 23 when the waist elastic member 23 contracts in the lateral direction (i.e., when the front waist portion 20 is in a natural state) (GH50 < d2). According to this configuration, the waist elastic member 23 that is contracted in the lateral direction is sandwiched between the welded portion pair 51 (expansion in the vertical direction is restricted). Thus, the position (movement) of the waist elastic member 23 in the lateral direction and vertical direction is restricted with respect to the skin side sheet 21 and the non-skin-side sheet 22.

Accordingly, even without fixing the waist elastic member 23 to the skin side sheet 21 and the non-skin-side sheet 22 using an adhesive, it is possible to suppress positional shifting of the waist elastic member 23 and maintain stretchability of the front waist portion. In other words, with the use of the welded portions 50, it is possible to attach the waist elastic member 23 to the skin-side sheet 21 and the non-skin-side sheet 22 without using an adhesive or with a reduced amount of an adhesive used. This makes it possible to suppress reduction in the flexibility of the front waist portion 20 (the stretchy sheet 40) caused by hardening of the adhesive. In addition, it is also possible to suppress the deterioration of the elasticity of the waist elastic members 23 caused by hardening of the adhesive.

Further, in the front waist portion 20, the waist elastic member 23 is fixed to the skin-side sheet 21 and the non-skin-side sheet at the side joining portions SS. Specifically, when the diaper 1 is manufactured, the side joining portions SS are formed in the state where the waist elastic member 23 in a stretched state is sandwiched between the skin-side sheet 21 and the non-skin-side sheet 22, and the end portions of the waist elastic member 23 are fixed to the side joining portions SS. Thus, even if the front waist portion 20 stretches significantly when the diaper 1 is putting on, it is possible to prevent the waist elastic member 23 from coming off from between the welded portion pair 51, thereby being able to maintain the elasticity of the front waist portion 20.

Further, the relationship between the space GH50 between the welded portion pair 51 and the outer diameters D1, D2 of the waist elastic member 23 is not limited to the above. For example, the waist elastic member 23 may be partially overlapped with the welded portion 50 to such an extent that the waist elastic member 23 does not break. In this case as well, the waist elastic member 23, in a state of being contracted in the lateral direction, is sandwiched between the welded portion pair 51, and the position of the waist elastic member 23 in the lateral direction and vertical direction is restricted with respect to the skin side sheet 21 and the non-skin-side sheet 22.

Furthermore, it is not necessarily the case that all of the waist elastic members 23 (elastic members) arranged in the front waist portion 20 (the stretchy sheet 40) are each sandwiched between the welded portion pair 51 to restrict their positions. It is sufficient that the position(s) of at least a part of the waist elastic members 23 is restricted by the welded portion pair(s) 51, and another part of the waist elastic members 23 may be fixed to the skin side sheet 21 and the non-skin-side sheet 22 with an adhesive.

### Spaced welded portion row Ra

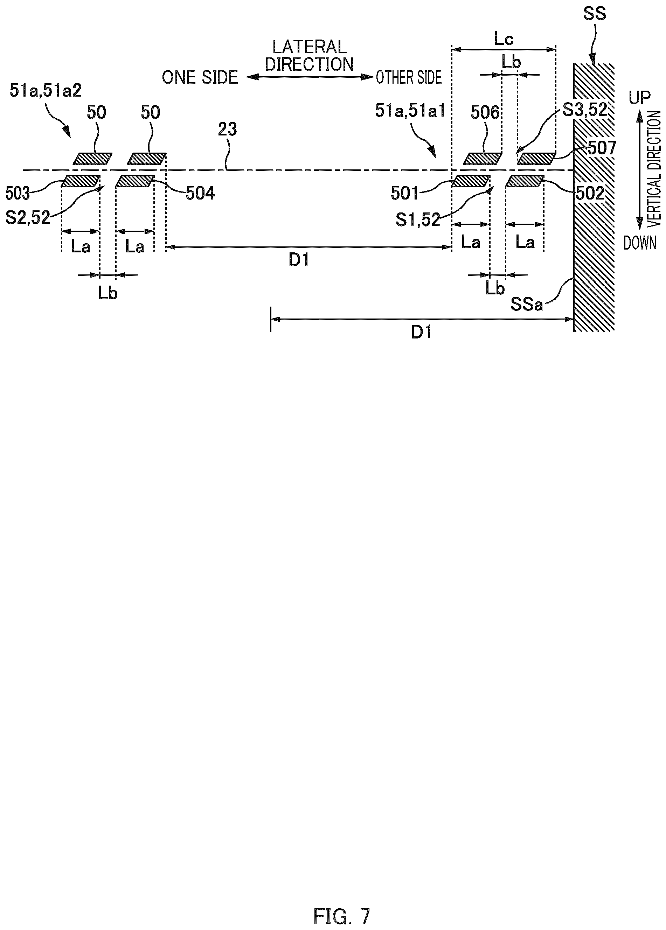

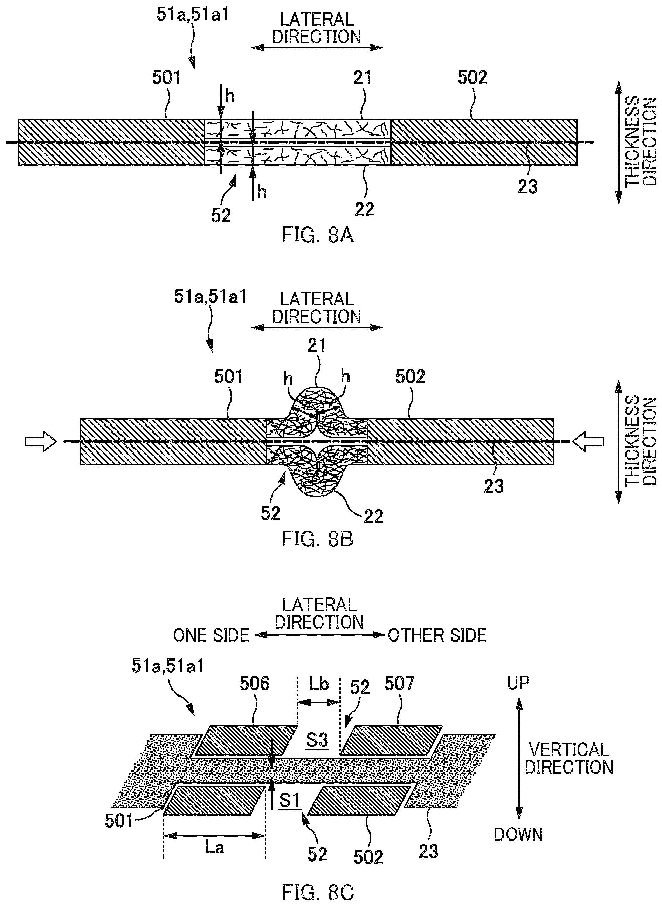

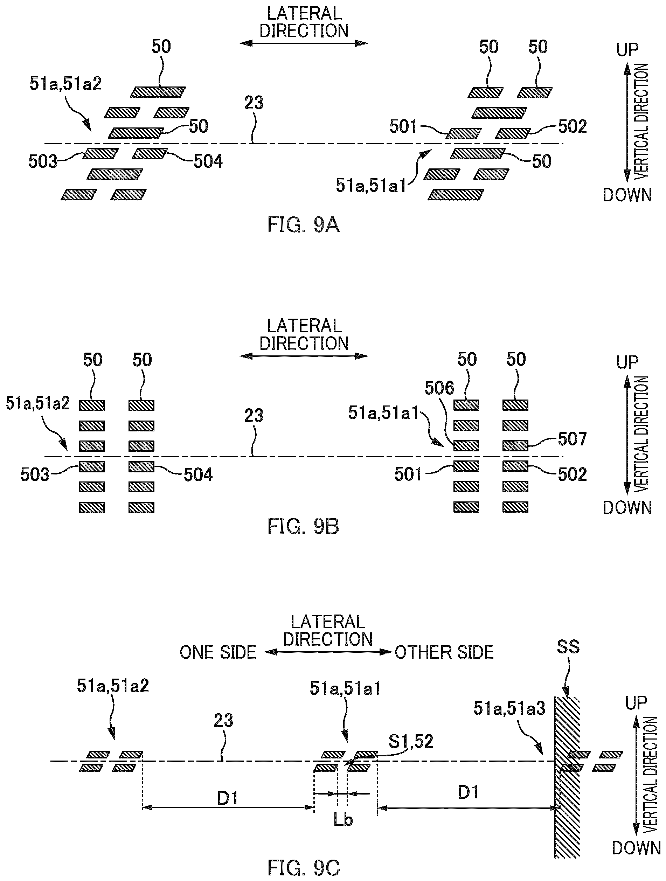

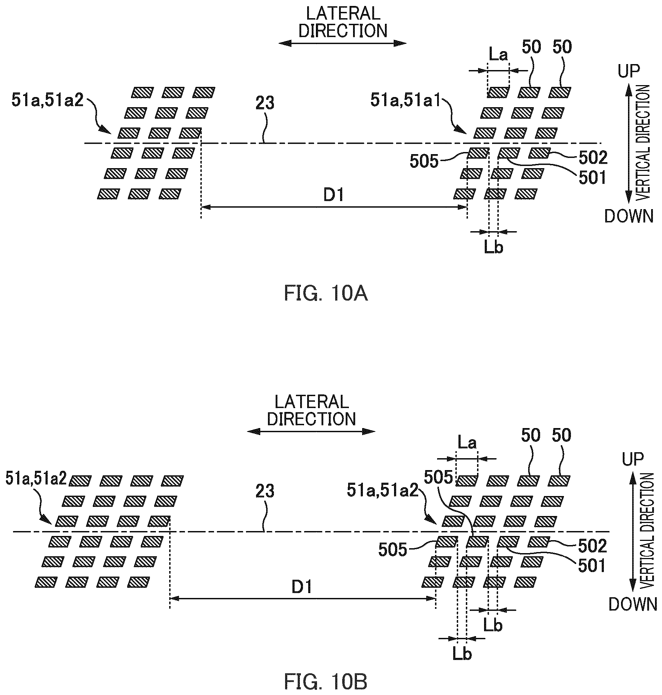

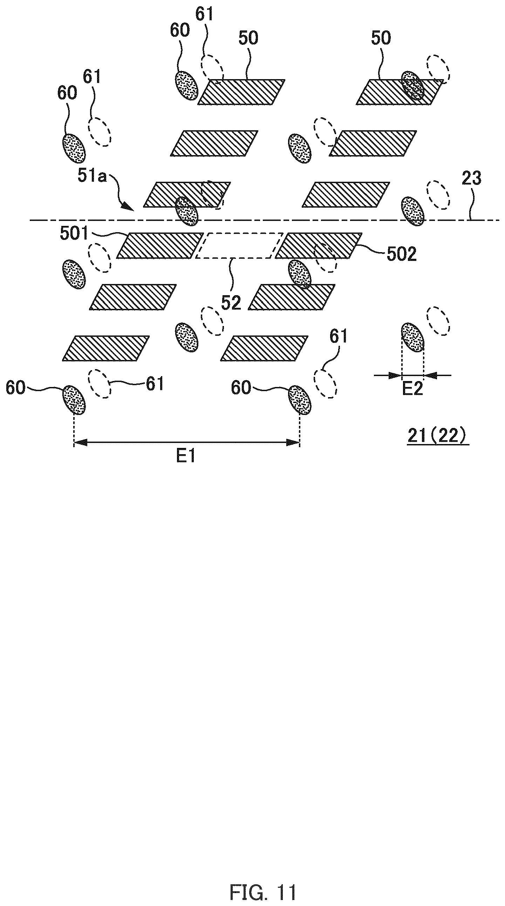

FIG. 7 is an illustrative diagram of the spaced welded portion pair 51a. FIG. 8A is an illustrative diagram of the spaced welded portion pair 51a in the stretched state, and FIGS. 8B and 8C are illustrative diagrams of the spaced welded portion pair 51a in the contracted state. FIGS. 9A to 9C, 10A, and 10B are illustrative diagrams of variations of the spaced welded portion pairs 51a. FIG. 11 is a diagram showing the relationship between a non-welded portions 52 and the pattern of compressed portions 60, 61 of the skin-side sheet 21 and the non-skin-side sheet 22.

As shown in FIGS. 4 and 6A, the front waist portion 20 (stretchy sheet 40) has two types of welded portion rows, which are the "spaced welded portion row Ra" and the "normal welded portion row Rb". In the front waist portion 20, multiple welded portion rows Ra, Rb are arranged in the lateral direction at predetermined intervals D1. Examples of the lateral intervals D1 of the welded portion rows Ra, Rb include about 4 to 14 times of a lateral length La of the welded portion 50, and the multiple welded portion rows Ra, Rb are arranged at relatively wide intervals.

Further, in the following description, among the welded portions 50 belonging to the spaced welded portion row Ra, the welded portion pair 51 that sandwich the waist elastic member 23 is referred to as "spaced welded portion pair 51a". In addition, among the welded portions 50 belonging to the normal welded portion row Rb, the welded portion pair 51 that sandwiches the waist elastic member 23 is referred to as "normal welded portion pair 51b".

As shown in FIG. 6A, in the normal welded portion row Rb, the welded portions 50 are arranged in a row in the vertical direction. Thus, as shown in FIG. 6B, the normal welded portion pair 51b includes two welded portions 50 arranged such that each one welded portion thereof is placed on each side of the waist elastic member 23 in the vertical direction.

Meanwhile, in the spaced welded portion row Ra, pairs of two welded portion 50 adjacent with a small space in the lateral direction are arranged in the vertical direction, to constitute two welded portion rows. Thus, as shown in FIG. 7, the spaced welded portion pair 51a includes four welded portions 50 arranged such that each two welded portions 50 thereof are placed on each side of the waist elastic member 23 in the vertical direction.

The spaced welded portion pair 51a will be specifically described with reference to FIG. 7. In FIG. 7, the spaced welded portion pair 51a located closer to the side joining portion SS is referred to as "first spaced welded portion pair 51a1 (first welded portion pair)". The spaced welded portion pair 51a located adjacent thereto on one side in the lateral direction is referred to as "second spaced welded portion pair 51a2 (second welded portion pair)". Both the first spaced welded portion pair 51a1 and the second spaced welded portion pair 51a2 sandwich the same waist elastic member 23 (first elastic member).

The first spaced welded portion pair 51a1 has a first welded portion 501 and a second welded portion 502 that are located on the same side (here, the lower side) in the vertical direction with respect to the waist elastic member 23, with a first space S1 therebetween in the lateral direction.

Then, in the state where the front waist portion 20 is stretched in the lateral direction, a lateral length D1 (first length) from the lateral one-side end of the first spaced welded portion pair 51a1 to the lateral other-side end of the second spaced welded portion pair 51a2 is longer than a lateral length Lb of the first space S1 (D1 > Lb).

In the following description, the length D1 is also referred to as "welded portion pair interval D1". Further, the lateral length Lb of the first space S1 is defined as a length from the lateral end on the other side (on the second welded portion 502 side) of the first welded portion 501 to the lateral end on the one side (on the first welded portion 501 side) of the second welded portion 502. The same applies to the length of other spaces (second space S2, third space S3) which will be described later.

As described above, in the spaced welded portion pair 51a, a part of the welded portion 50 is hollowed out, on one side of the waist elastic member 23 in the vertical direction, such that a non-welded portion 52 is formed between welded portions (501 and 502) that are arranged side-by-side in the lateral direction. This non-welded portion 52 corresponds to a region having a length that is about the same as the lateral length Lb of the first space S1 and is narrower than the welded portion pair interval D1. Thus, when the waist elastic member 23 contracts (FIG. 8B), the two welded portions (501 and 502) on outer sides of the non-welded portion 52 come closer to each other, and the skin-side sheet 21 and the non-skin-side sheet 22 (hereinafter also referred to as "sheets 21, 22") located in the non-welded portion 52 are sandwiched between the two welded portions (501 and 502) . As a result, the density of the fibers constituting the sheets 21 and 22 increases in the non-welded portion 52.

As shown in FIG. 6B, a non-welded portion is also formed between the normal welded portion pair 51b arranged in the lateral direction. However, this non-welded portion is as wide as the welded portion pair interval D1. Thus, even if the waist elastic member 23 contracts, it is difficult for the welded portions 50 to come closer to each other to an extent that the fiber density of the sheets 21 and 22 in the non-welded portion increases. Further, if the length (D1) of the sheets 21, 22 located in the non-welded portion is long, the sheets 21, 22 are more likely to bend on the outer side in the thickness direction with respect to the welded portions 50. Accordingly, the sheets 21, 22 cannot be sandwiched between the welded portions 50, which makes it difficult to increase the fiber density of the sheets 21 and 22.

Thus, it is preferable to provide the non-welded portion 52 corresponding to the space Lb, which is smaller than the welded portion pair interval D1, as in the spaced welded portion pair 51a. Specifically, Lb is preferably equal to 2.54 mm or less, and more preferably is about 0.5 mm. According to this configuration, when the waist elastic member 23 contracts, the sheets 21, 22 in the non-welded portion 52 can be sandwiched between the welded portions (501 and 502), thereby being able to increase the fiber density of the sheets 21, 22 in the non-welded portion 52. As a result, in the non-welded portion 52, the dense fibers are more likely to be entangled with the waist elastic member 23, thereby being able to restrict the movement of the waist elastic member 23. Accordingly, it can be suppressed that the waist elastic member 23 comes out of the spaced welded portion pair 51a.

In particular, in the case of the pants-type diaper 1, in the process of forming the side joining portions SS in the manufacturing of the diaper 1, the sheets 21, 22 are welded relatively strongly, and thus the waist elastic member 23 may break. Further, while the diaper 1 is being put on, a strong pulling force is applied to the side joining portions SS when the diaper 1 is being pulled up with the side joining portions SS being grasped, which may cause the waist elastic member 23 to break. Even if the waist elastic member 23 is separated from the side joining portions SS, the waist elastic member 23 contracting from the side joining portions SS is restricted by the spaced welded portion pair 51a, thereby being able to suppress the case where the waist elastic member 23 comes out of the spaced welded portion pair 51a. Thus, reduction in the stretchability of the front waist portion 20 can be suppressed.

Further, even in the configuration of the normal welded portion pair 51b (FIG. 6B), with an increase in the lateral length of the welded portions 50, the frictional force between the welded portions 50 and the waist elastic member 23 increases, thereby being able to suppress the case where the waist elastic member 23 comes out of the normal welded portion pair 51b. However, if the stiff welded portions 50 are lengthened, the flexibility of the front waist portion 20 will decrease.

In contrast, in the spaced welded portion pair 51a, the non-welded portion 52 is provided at a part of the pair 51a in the lateral direction. Accordingly, as compared to the case where the normal welded portion pair 51a of the same length Lc as that of the spaced welded portion pair 51a is provided, it is possible to reduce the area ratio of the welded portion 50 in the lateral direction, thereby being able to ensure the flexibility of the front waist portion 20. Further, the non-welded portions 52 are more likely to become folding base points in the lateral folding of the front waist portion 20. Thus, the front waist portion 20 easily bends in the lateral direction, and is more likely to fit around the wearer's waist.

As has been described above, with provision of the spaced welded portion pair 51a in the front waist portion 20 (stretchy sheet 40), it is possible to suppress the case where the waist elastic member 23 comes out thereof, suppress reduction in the stretchability, and ensure the flexibility of the front waist portion 20. In other words, with the restriction of the position of the waist elastic member 23 with respect to the sheets 21, 22 by the welded portion pair 51 and the reduction in the amount of adhesive used, it is possible to ensure the flexibility of the front waist portion 20.

In the front waist portion 20 illustrated in FIG. 4, the spaced welded portion rows Ra (the spaced welded portion pairs 51a) of a shape meandering in the lateral direction are arranged in the vicinity of the side joining portions SS and in the vicinity of the side portions of the absorbent main body 10. However, the shape, number and arrangement position of the spaced welded portion rows Ra are not particularly limited, as long as the front waist portion 20 (stretchy sheet 40) has at least one spaced welded portion pair 51a. Further, although the shape of the welded portions 50 is a parallelogram in FIG. 7, the shape of the welded portions 50 is not particularly limited. Further, the shapes of the multiple welded portions 50 in each of the welded portion rows Ra, Rb may be the same or different.

Further, all of the welded portion rows provided in the front waist portion 20 may be the spaced welded portion row Ra, but it is preferable to mix the normal welded portion row Rb, which is constituted by a single row, with the spaced welded portion row Ra. According to this configuration, the flexibility of the front waist portion 20 is ensured while suppressing the case where the waist elastic member 23 comes out of the welded portion pair 51.

Further, in the state where the front waist portion 20 is stretched in the lateral direction, it is preferable that the lateral length Lb of the first space S1 of the first spaced welded portion pair 51a1 is less than or equal to the lateral length La of the first welded portion 501, and is less than or equal to the lateral length La of the second welded portion 502 (Lb ≤ La).

According to this configuration, the welded portions (501, 502) increase in stiffness, thereby being able to sandwich the non-welded portion 52 therebetween. As a result, the fiber density of the sheets 21, 22 increases in the non-welded portion 52, thereby being able to entangle the fibers with the waist elastic member 23. Further, the longer the welded portions (501, 502), the larger the frictional force generated between the waist elastic member 23 and the welded portions (501, 502), thereby enabling the welded portions (501, 502) to restrict the movement of the waist elastic member 23. This can suppress the case where the waist elastic member 23 comes out of the spaced welded portion pair 51a.

However, there is no limitation to the above configuration, the lateral length Lb of the first space S1 may be longer than the lateral lengths La of the first welded portion 501 and the second welded portion 502. Further, the lateral lengths La of the first welded portion 501 and the second welded portion 502 may be the same or different.

Further, in the state where the front waist portion 20 is stretched in the lateral direction, it is preferable that the welded portion pair interval D1 is longer than the lateral length Lc of the entire first spaced welded portion pair 51a1 (D1 > Lc).

According to this configuration, the interval between the welded portion rows Ra and Rb increases, thereby being able to suppress the case where the welded portions 50 are densely arranged in the lateral direction and ensure the flexibility of the front waist portion 20. In other words, in the first spaced welded portion pair 51a1, it can be said that the individual lateral lengths of the multiple welded portions (501, 502) and the non-welded portions 52 are short. Accordingly, with the short welded portions (501, 502), it is possible to ensure the flexibility of the front waist portion 20, and in the short non-welded portion 52, it is possible to increase the fiber density of the sheets 21, 22. However, there is no limitation to the above configuration, the welded portion pair interval D1 may be less than or equal to the lateral length Lc of the first spaced welded portion pair 51a1.

Further, as shown in FIG. 7, it is preferable that the welded portion pair (the second spaced welded portion pair 51a2) adjacent in the lateral direction to the first spaced welded portion pair 51a also has the same configuration, and may have a third welded portion 503 and a fourth welded portion 504 located on the same side (here, the lower side) in the vertical direction with respect to the waist elastic member 23, with the second space S2 therebetween in the lateral direction. Then, in the state where the front waist portion 20 is stretched in the lateral direction, it is preferable that the welded portion pair interval D1 is longer than the lateral length Lb of the second space S2 (D1 > Lb).

With the spaced welded portion pairs 51a being arranged consecutively in the lateral direction as such, it is possible to suppress the case where the waist elastic member 23 comes out of the welded portion pair 51 more reliably, thereby being able to suppress a reduction in the stretchability of the front waist portion 20. However, there is no limitation to the above configuration, the welded portion pair (the second welded portion pair) adjacent to the first spaced welded portion pair 51a in the lateral direction may be the normal welded portion pair 51b (FIG. 6B) .

Further, as in the first spaced welded portion pair 51a1 shown in FIGS. 10A and 10B, there may be one or more fifth welded portions 505 aligned with the first welded portion 501 and the second welded portion 502 on the same side in the vertical direction with respect to the waist elastic member 23, with a space in the lateral direction. In other words, three or more welded portions 50 may be provided on the same side in the vertical direction with respect to the waist elastic member 23.

Then, in the state where the front waist portion 20 is stretched in the lateral direction, it is preferable that the welded portion pair interval D1 is longer than the lateral length Lb (FIGS. 10A and 10B) of the space between the fifth welded portion 505 and the first welded portion 501 adjacent to the fifth welded portion 505 in the lateral direction, and is also longer than the lateral length Lb (FIG. 10B) of the space between the fifth welded portions 505 that are adjacent to each other in the lateral direction (D1 > Lb).

In this case, two or more non-welded portions 52 that are narrow regions are formed on one side of the waist elastic member 23 in the vertical direction. Thus, the waist elastic member 23 is more likely to be entangled with the fibers at two places, which further suppresses the case where the waist elastic member 23 comes out of the spaced welded portion pair 51a.

Further, in the above case as well, in the state where the front waist portion 20 is stretched in the lateral direction, the lateral length Lb of the space between the fifth welded portion 505 and the first welded portion 501 or the fifth welded portion 505 adjacent to the above fifth welded portion 505 in the lateral direction may be less than or equal to the lateral length La of each of the welded portions (the first welded portion 501, the second welded portion 502, fifth welded portion 505) (Lb ≤ La). According to this configuration, the welded portions (501, 502, 505) that are long in the lateral direction can firmly sandwich the non-welded portion 52 that is short in the lateral direction, thereby being able to increase the fiber density in the non-welded portion 52.

Up to this point, we have focused on one side (the lower side) in the vertical direction with respect to the waist elastic member 23, but it is preferable that the non-welded portion 52 is also provided on the side opposite thereto. In other words, it is preferable that the first spaced welded portion pair 51a1 may have a sixth welded portion 506 and a seventh welded portion 507 located with a third space S3 in the lateral direction on the opposite (upper) side in the vertical direction with respect to the waist elastic member 23. Then, in the state where the front waist portion 20 is stretched in the lateral direction, it is preferable that the welded portion pair interval D1 is longer than the lateral length Lb of the third interval S3 (D1 > Lb).

With the non-welded portions 52, which are narrow regions, being located on both sides of the waist elastic member 23 in the vertical direction, the non-welded portions 52 easily contract along the lateral direction, and the fiber density of the sheets 21, 22 is more likely to increase in the non-welded portions 52. Further, the fibers are more likely to be entangled with the elastic member from both sides in the vertical direction. As a result, the fibers are more likely to be entangled with the waist elastic member 23, thereby being able to further suppress the case where the waist elastic member 23 comes out of the spaced welded portion pair 51a.

However, there is no limitation to the above configuration, for example, as in a variation in FIG. 9A, the welded portion 50 on one side in the vertical direction does not have a space therein and does not have to have the non-welded portion 52. In this case, the lateral length of the welded portion 50 without the non-welded portion 52 may be shorter than or equal to the total length of the welded portions 501, 502 and the non-welded portion 52 on the opposite side (FIG. 9A). Further, in the spaced welded portion pairs 51a1, 51a2 adjacent to each other in the lateral direction, the non-welded portions 52 may be located on the sides opposite to each other therebetween (FIG. 9A) or may be located on the same side.

Further, as shown in FIG. 8C, in the state where the front waist portion 20 is stretched in the lateral direction, it is preferable that the first space S1 between the first welded portion 501 and the second welded portion 502 on the lower side with respect to the waist elastic member 23 and the third space S3 between the sixth welded portion 506 and the seventh welded portion 507 on the upper side have an overlapping region in the lateral direction.

According to this configuration, in the region in which the first space S1 and the third space S3 overlap with each other, the contraction of the non-welded portion 52 is less likely to be inhibited by the welded portions 50 located on the side opposite thereto in the vertical direction, so that the non-welded portion 52 is able to contract firmly. Thus, in the non-welded portion 52, the fiber density of the sheets 21, 22 is more likely to increase, thereby being able to further suppress the case where the waist elastic member 23 comes out of the spaced welded portion pair 51a.

Further, it is preferable that the lateral center of the first space S1 is shifted in the lateral direction from the lateral center of the third space S3. According to this configuration, in the region in which the first space S1 and the third space S3 do not overlap in the lateral direction, as shown in FIG. 8C, a part of the waist elastic member 23 faces a welded portion (e.g., the sixth welded portion 506) on one side (e.g., the upper side) in the vertical direction, and faces the non-welded portion 52 on the other side (e.g., the lower side). Thus, the waist elastic member 23 is subjected to a force toward the vertical center (downward) from the welded portion (506), while also being subjected to a force toward the vertical center (upward) from the concentrated and overflowing fibers in the non-welded portion 52. Thus, the fibers of the sheets 21, 22 become more likely to be entangled with the waist elastic member 23, thereby being able to further suppress the case where the waist elastic member 23 comes out of the spaced welded portion pair 51a.

Further, as shown in FIG. 6A, the welded portions 50 that are arranged to be spaced apart in the vertical direction from the first welded portion 501 is referred to as eighth welded portion 508, and the welded portions 50 that are arranged to be spaced apart from the second welded portion 502 in the vertical direction is referred to as ninth welded portion 509. The eighth welded portion 508 may be shifted in the lateral direction from the first welded portion 501 or another eighth welded portion 508, the first welded portion 501 and the other eighth welded portion 508 being adjacent to the above eighth welded portion 508 in the vertical direction. The ninth welded portion 509 may be shifted in the lateral direction from the second welded portion 502 or another ninth welded portion 509, the second welded portion 502 and the other ninth welded portion 509 being adjacent to the above ninth welded portion 509 in the vertical direction. Then, in the state where the front waist portion 20 is stretched in the lateral direction, it is preferable that the welded portion pair interval D1 is longer than the lateral length Lb of the space between the eighth welded portion 508 and the ninth welded portion 509 adjacent in the lateral direction (D1 > Lb).

As such, the welded portions (508, 509) arranged side-by-side in the vertical direction are shifted in the lateral direction from each other, and thus a portion of the waist elastic member 23 located in the non-welded portion 52 overlaps, in the lateral direction, not only with the welded portions 50 that sandwich the waist elastic member 23, but also with the welded portions (508, 509) arranged side-by-side in the vertical direction, and is susceptible to the influence of the welded portions (508, 509) as well. For example, the concentrated fibers of the non-welded portion 52 are pushed toward the waist elastic member 23 by the welded portions (508, 509). As a result, the fibers are more likely to become entangled with the waist elastic member 23 in the non-welded portion 52, which further suppresses the case where the waist elastic member 23 comes out of the spaced welded portion pair 51a.

However, there is no limitation to the above configuration, as shown in FIG. 9B, the welded portions 50 adjacent to each other in the vertical direction may be aligned in a straight line along the vertical direction without being shifted in the lateral direction.

Furthermore, the pair of sheets 21, 22 sandwiching the waist elastic member 23 may each have compressed portions 60, 61 (embossed pattern) obtained by compressing the single sheet in the thickness direction. In the compressed portions 60, 61, the fibers constituting the sheets 21, 22 are tightly entangled, and few fibers can move freely.

Thus, when each of the pair of sheets 21, 22 has multiple compressed portions 60, 61, the following is preferable. As shown in FIG. 11, in a plan view in the thickness direction of the front waist portion 20 stretched in the lateral direction, it is preferable that at least a part of the multiple compressed portions 60 in one sheet (e.g., the skin-side sheet 21) has a region that does not overlap with the compressed portions 61 in the other sheet (e.g., the non-skin-side sheet 22). In other words, it is preferable that a part or all of the multiple compressed portions 60 of the skin-side sheet 21 are overlapped with the compressed portions 61 of the non-skin-side sheet 22. The compressed portions 60, 61 may be partially overlapped or may not be completely overlapped with each other at all.

This configuration increases the probability that even if the compressed portion 60 of one sheet (e.g., the skin-side sheet 21) is located in the non-welded portion 52, the compressed portion 61 of the other sheet (e.g., the non-skin-side sheet 22) is not located therein. Thus, the waist elastic member 23 can be entangled with the unpressed fibers of at least one of the sheets in the non-welded portion 52. This can more reliably suppress the case where the waist elastic member 23 comes out of the spaced welded portion pair 51a.

Further, when at least one of the pair of sheets 21, 22 has the multiple compressed portions 60, 61, the following is preferable. In the plan view in the thickness direction of the front waist portion 20 in the laterally stretched state, it is preferable that the first space S1 between the first spaced welded portion pair 51a1 shown in FIG. 7 has a region that is not overlapped with the compressed portions 60, 61 of at least one of the sheets.

According to this configuration, the non-welded portion 52 is not overlapped with the compressed portions 60, 61, and the uncompressed free fibers are located in the non-welded portion 52. Thus, the fibers of the sheets 21, 22 are more likely to be entangled with the waist elastic member 23, which can suppress the case where the waist elastic member 23 comes out of the spaced welded portion pair 51a.

When at least one of the pair of sheets 21, 22 has the multiple compressed portions 60, 61, it is preferable that the lateral length Lb of the first space S1 between the first spaced welded portion pair 51a1 shown in Fig. 7 is equal to or less than the maximum value E1 of a lateral interval between the compressed portions 60, 61 adjacent in the lateral direction (Lb ≤ E1), in the state where the front waist portion 20 is stretched in the lateral direction. Note that the interval between the compressed portions 60, 61 adjacent in the lateral direction is defined as an interval between the compressed portions 60, 61 adjacent in the lateral direction among the compressed portions 60, 61 provided at the same sheet (21 or 22).

According to this configuration, the non-welded portion 52 is more likely to be fit in between the compressed portions 60, 61 adjacent in the lateral direction, and uncompressed free fibers are more likely to be located in the non-welded portion 52. Thus, the fibers of the sheets 21, 22 are more likely to be entangled with the waist elastic member 23, thereby being able to suppress the case where the waist elastic member 23 comes out of the spaced welded portion pair 51a.

Further, the lateral length Lb of the first space S1 may be larger than the maximum value E2 of the lateral length of the compressed portions 60, 61 (Lb > E2). According to this configuration, even if the compressed portion 60, 61 results in being positioned at the non-welded portion 52, a region that does not overlap with the compressed portions 60, 61 is formed in the non-welded portion 52. Thus, the fibers of the sheets 21, 22 are more likely to be entangled with the waist elastic member 23, thereby being able to suppress the case where the waist elastic member 23 comes out of the spaced welded portion pair 51a.

However, there is no limitation to the above configuration, for example, the sheets 21, 22 may not have the compressed portions 60, 61. Further, in FIG. 11, the compressed portions 60, 61 of an oval shape are arranged in the lateral direction and the vertical direction at regular intervals. However, the shape, number, arrangement, and the like of the compressed portions 60, 61 are not particularly limited, and for example, the compressed portions 60, 61 having shapes different between the skin-side sheet 21 and the non-skin-side sheet 22 may be provided.

Further, the first space S1 between the first spaced welded portion pair 51a1 is preferably as follows. First, as shown in Fig. 8A, the length of twice the thickness h of at least one of the pair of sheets 21, 22 that sandwich the waist elastic member 23 is defined as a second length 2h. In this event, in the state in which the front waist portion 20 is stretched in the lateral direction, it is preferable that the lateral length Lb of the first space S1 is equal to or more than the second length 2h (Lb ≥ 2h).

According to this configuration, it is possible to prevent the non-welded portion 52 from not being contracted in the lateral direction due to the first space S1 being too narrow. With the above configuration, as shown in FIG. 8B, the sheets 21, 22 in the non-welded portion 52 are sandwiched between the welded portions (501, 502) while being bent outward in the thickness direction, thereby increasing the fiber density of the sheets 21, 22. Thus, the fibers of the sheets 21, 22 are more likely to be entangled with the waist elastic member 23, thereby being able to suppress the case where the waist elastic member 23 comes out of the spaced welded portion pair 51a.

Note that a method of measuring the thicknesses of sheets 21 and 22 can be performed by a known method. For example, in the spaced welded portion pair 51a, the sheets 21, 22 are cut along the lateral direction between the welded portions arranged side-by-side in the vertical direction (e.g., between the welded portions 501, 502 and the welded portions 506, 507). This cut surface is observed using a scanning electron microscope (e.g., HITACHI FlexSEM 1000, or equivalent thereto) in the state where the cut surface is stretched in the lateral direction as shown in FIG. 8A., thereby being able to perform measurements.

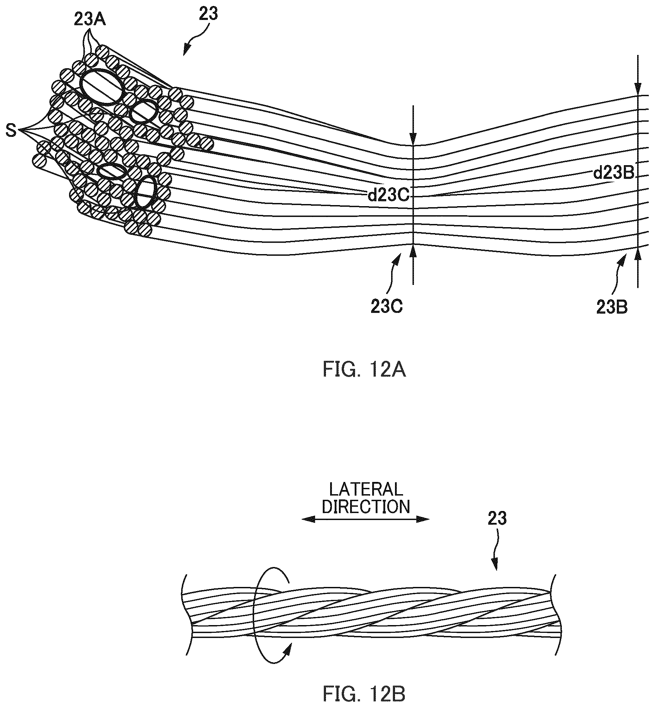

FIGS. 12A and 12B are illustrative diagrams of the waist elastic member 23. As shown in FIG. 12A, the waist elastic member 23 is preferably a string-like elastic member in which multiple elastic fibers 23A are assembled. According to this configuration, even if a part of the elastic fibers 23A break during the process of forming the side joining portions SS, the remaining elastic fibers 23A are connected, thereby being able to prevent the waist elastic members 23 from breaking. Thus, it is possible to suppress the case where the waist elastic member 23 comes out of the welded portion pair 51, thereby being able to ensure the stretchability of the front waist portion 20.

Further, since the waist elastic member 23 is constituted by the multiple elastic fibers 23A, spaces S are more likely to be formed between the elastic fibers 23A. Thus, even if the waist elastic member 23 is compressed during a process of forming the side joining portions SS (welded portion, etc.), and the like, the spaces S are compressed to provide escape spaces (the elastic member 23 is not severed by being compressed by the protruding pattern forming the side joining portions SS), thereby being able to suppress the case where the waist elastic member 23 breaks.

Further, since the waist elastic member 23 is constituted by the multiple elastic fibers 23A, the thickness of the waist elastic member 23 is more likely to partially vary depending on how the elastic fibers 23A are assembled. Thus, it is preferable that the elastic member 23 has a large diameter portion (first outer diameter portion) 23B having an outer diameter of a first outer diameter d23B, and a small diameter portion (second outer diameter portion) 23C having an outer diameter d23C smaller than the first outer diameter d23B.

According to this configuration, when the large diameter portion 23B of the elastic member 23 is positioned between the spaced welded portion pair 51a, the large-diameter portion 23B is susceptible to the force from the fibers that overflow when the non-welded portion 52 contracts, and the fibers more likely to be entangled therewith. In addition, the force of the large-diameter portion 23B to expand is strongly applied to the welded portions 50, and the large-diameter portion 23B is tightly sandwiched between the welded portions 50. This makes it possible to further suppress the case where the waist elastic member 23 comes out of the spaced welded portion pair 51a.

Further, it is preferable that the waist elastic member 23 in a twisted state is arranged between the pair of sheets 21, 22. According to this configuration, when the waist elastic member 23 contracts, the pitch of the twists of the waist elastic member 23 decreases, so that the fibers of the sheets 21, 22 are more likely to be entangled with the twisted portion of the waist elastic member 23 in the non-welded portion 52. Thus, it is possible to further suppress the case where the waist elastic member 23 comes out of the spaced welded portion pair 51a.

Whether the waist elastic member 23 is in a twisted state can be visually confirmed, for example, when the pair of sheets 21, 22 constituting the front waist portion 20 are separated such that the waist elastic members 23 are exposed.

Further, the first spaced welded portion pair 51a1 is adjacent to the second spaced welded portion pair 51a2 on one side in the lateral direction, with the relatively wide welded portion pair interval D1. As shown in FIG. 9C, it is preferable that the first spaced welded portion pair 51a1 is adjacent to the welded portion pair 51a3 (third welded portion pair) on the other side in the lateral direction, with the relatively wide interval (D1) as well. In other words, it is preferable that the lateral length D1 from the lateral other-side end of the first spaced welded portion pair 51a1 to the lateral one-side end of the welded portion pair 51a3 is longer than the lateral length Lb of the first space S1 (D1 > Lb).

According to this configuration, it is possible to prevent the welded portion pairs 51 from being closely arranged in the lateral direction, thereby ensuring the flexibility of the front waist portion 20. Note that the welded portion pair 51a3 that is adjacent thereto on the other side in the lateral direction may be the spaced welded portion pair 51a as shown in FIG. 9C or may be the normal welded portion pair 51b. Further, assuming that the welded portion pair 51 that is a first one from the side joining portion SS is the spaced welded portion pair 51a (first welded portion pair), the welded portion pair 51a3 (third welded portion pair) adjacent thereto on the outer side in the lateral direction (the other side in FIG. 9C) may be located at the side joining portion SS, as shown in FIG. 9C. Furthermore, when the first spaced welded portion pair 51a1 is located close to the side joining portion SS, as shown in FIG. 7, there does not have to be any welded portion adjacent to the first spaced welded portion pair 51a1 (first welded portion pair) on the outer side in the lateral direction (the other side in FIG. 7), or the second spaced welded portion pair 51a2 that is a second one from the side joining portion SS may correspond to the first welded portion pair.

Further, as described above, when the side joining portions SS are formed or when the side joining portions SS are grasped and pulled up, the waist elastic member 23 is more likely to break. Accordingly, the waist elastic member 23 is more likely to come out of the welded portion pair 51 in the vicinity of the side joining portions SS. Thus, as shown in FIG. 7, in the state where the front waist portion 20 is stretched in the lateral direction, it is preferable that at least a part of the spaced welded portion pair 51a (the first spaced welded portion pair 51a1) is located in a region on the inner side in the lateral direction with respect to the lateral inner end SSa of the side joining portion SS by the welded portion pair interval D1.

According to this configuration, the welded portion pair 51 closest to the side joining portion SS is more likely to be the spaced welded portion 51a. Thus, even if the waist elastic member 23 is severed at the side joining portion SS, the spaced welded portion pair 51a can suppress the case where the waist elastic member 23 comes out in the vicinity of the side joining portions SS. Thus, the stretchability is maintained over a wide range in the lateral direction of the front waist portion 20.

Furthermore, by arranging multiple (here, four) spaced welded portion rows Ra in the lateral direction in the vicinity of the side joining portions SS, as shown in FIG. 4, it is possible to more reliably suppress the case where the waist elastic member 23 comes out of the spaced welded portion pair 51a in the vicinity of the side joining portions SS. However, there is no limitation to the above configuration, and the spaced welded portion pair 51a may be located at a position more distant from the inner end SSa of the side joining portion SS than the position distant therefrom by the welded portion pair interval D1, or the welded portion pair 51 closest to the side joining portion SS may be the normal welded portion pair 51b.

Further, as shown in FIG. 4, the waist elastic member 23D that overlaps with the absorbent main body 10 may have a non-continuous portion SB in which the waist elastic member 23D is cut and non-continuous in the lateral central portion of the diaper 1, and continuous portions SA located on the outer side in the lateral direction with respect to the non-continuous portion SB. In the region corresponding to the non-continuous portion SB, stretchability is hardly exhibited, and the stretching force of the waist elastic member 23 per vertical unit width is lower than in the regions corresponding to the continuous portions SA. This suppresses the case where the absorbent main body 10 contracts due to the waist elastic member 23, and the flatness of the absorbent main body 10 (particularly, the absorbent core 11) is ensured, which improves the fit to the wearer. Further, when the absorbent main body 10 has a graphic (not shown) visible from the non-skin side, the visibility of the graphic is improved

When the waist elastic member 23 is cut during the process of manufacturing the diaper 1, the waist elastic member 23 increases in its diameter while contracting, and is sandwiched between the welded portion pair 51 in the middle. Accordingly, in the state where the front waist portion 20 is stretched, the cut end portion of the waist elastic member 23 in a stretched state is held by the welded portion pair 51, thereby ensuring stretchability in the continuous portions SA. Note that when the waist elastic member 23 is cut at multiple locations, the waist elastic member 23D (not shown) that does not exhibit stretchability is located at the non-continuous portion SB.

Then, it is preferable to provide the spaced welded portion pair 51a (first welded portion pair) having the same configuration as the first spaced welded portion pair 51a1 (FIG. 7), at the end on the central side in the lateral direction of each of the regions (SA) in which stretchability is desired to be exerted. For example, as shown in FIG. 4, the spaced welded portion rows Ra are provided in the vicinity of the side ends of the absorbent main body 10. Then, it is preferable that the spaced welded portion pair 51a sandwiches the end portion of the waist elastic member 23D on the central side in the lateral direction of each of the continuous portions SA. According to this configuration, the fibers in the non-welded portion 52 are more likely to be entangled with the cut end portion of the waist elastic member 23D, which makes it possible to suppress the case where the waist elastic member 23D comes out of the spaced welded portion pair 51a. This ensures the stretchability of the front waist portion 20.

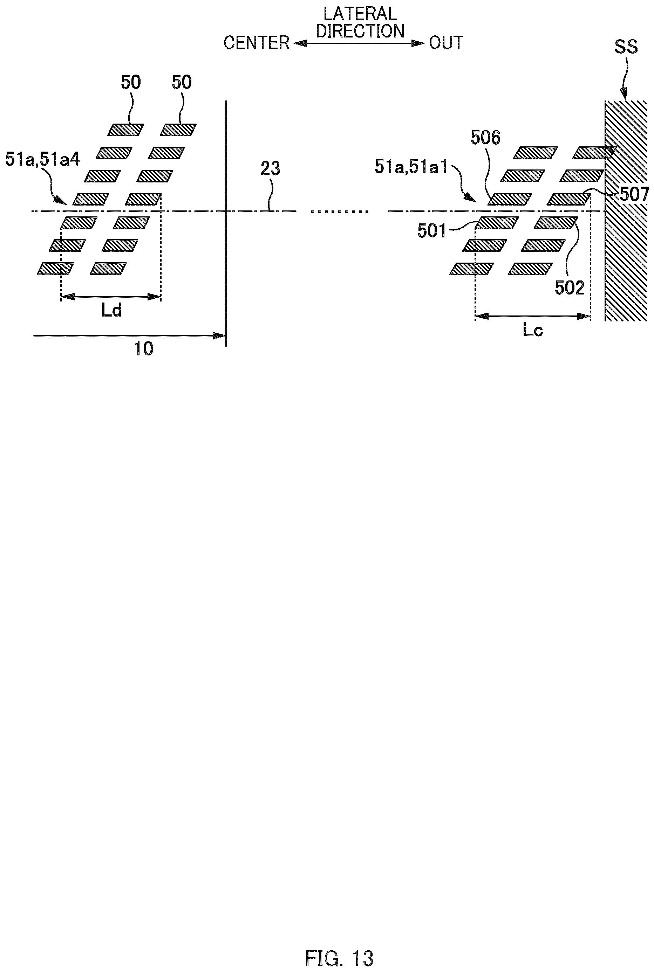

FIG. 13 is an illustrative diagram of the spaced welded portion pairs 51a provided at the side portion of the absorbent main body 10 and in the vicinity of the side joining portion SS. The first spaced welded portion pairs 51a1 (first welded portion pairs) described with reference to FIG. 7 are located at the lateral ends of the front waist portion 20 (the diaper 1), and the spaced welded portion pairs 51a located at the lateral central portion of the front waist portion 20 (the diaper 1) are referred to as fourth spaced welded portion pairs (fourth welded portion pairs).

The fourth spaced welded portion pair 51a4 also, as with the first spaced welded portion pair 51a1, has the multiple welded portions 50 spaced apart in the lateral direction on the same side in the vertical direction with respect to the waist elastic member 23. However, it is preferable that the lateral length Lc of the first spaced welded portion pair 51a1 is longer than the lateral length Ld of the fourth spaced welded portion pair 51a4 (Lc > Ld).

The lateral length Lc of the first spaced welded portion pair 51a being long means that the length of the welded portions (501, 502) is long and/or the number of the non-welded portions 52 is large. Thus, the waist elastic member 23 is less likely to come out of the first spaced welded portion pair 51a1, as compared with the fourth spaced welded portion pair 51a4. Accordingly, although the waist elastic member 23 is more likely to be severed at the side joining portion SS, it is possible to effectively suppress the case where the waist elastic member 23 comes out of the first spaced welded portion pair 51a1 that is close to the side joining portion SS. Meanwhile, in the lateral central portion of the front waist portion 20, in which the waist elastic member 23 is less likely to be severed as compared with the side joining portions SS, the fourth spaced welded portion pair 51a4, which has a shorter length, suppresses the case where the waist elastic member 23 comes out while ensuring the flexibility of the front waist portion 20. However, there is no limitation to the above configuration, and the lateral length of the spaced welded portion pairs 51a may be constant.

The embodiment in which the stretchy sheet 40 is provided to the front waist portion 20 has been described. Accordingly, the stretchy sheet 40 is provided to the front waist portion 20 so that the lateral direction of the stretchy sheet 40 (the direction along which the elastic members extend) is aligned with the lateral direction of the underpants-shaped diaper 1, and the vertical direction of the stretchy sheet 40 (the direction perpendicular to the elastic members) is aligned with the vertical direction of the underpants-shaped diaper 1.

Further, in the above embodiment, both the front waist portion 20 and the back waist portion 30 are configured with the stretchy sheet 40, but only one of the waist portions may be configured with the stretchy sheet 40. Further, as in the back waist portion 30, when an extension portion 30E is provided below the side joining portions SS, and elastic members are arranged along the lateral direction in the extension portion 30E as well, which is different from FIG. 2, the extension portion 30E may also be configured with the stretchy sheet 40.

### Second Embodiment

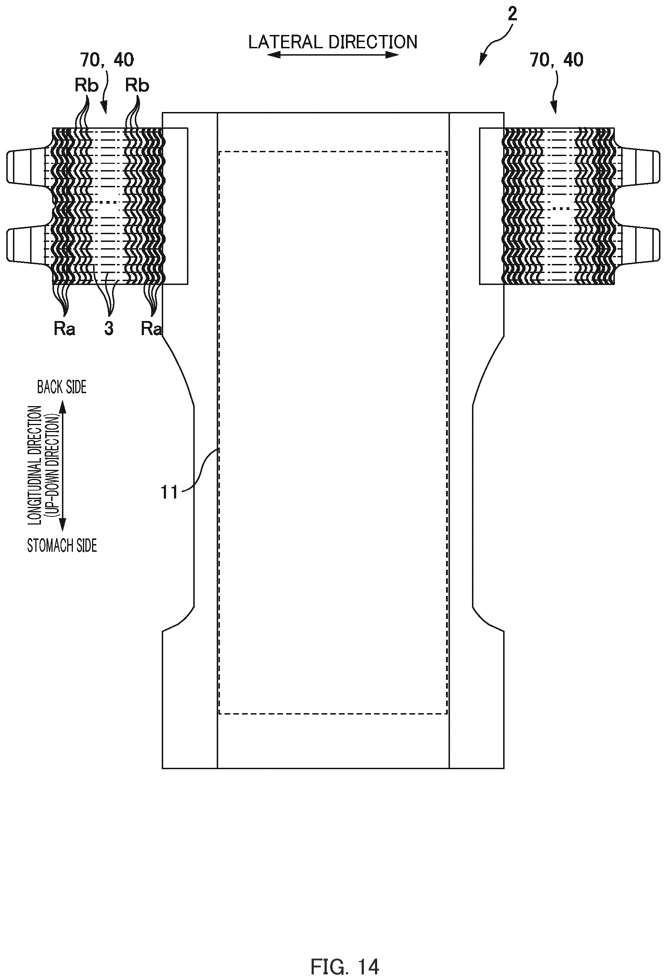

FIG. 14 is a plan view of a tape-type diaper 2 in a state of being unfolded in the longitudinal direction. The first embodiment gives an example in which the stretchy sheet 40 is provided to the waist portions 20, 30 of a pants-type diaper 1 for adults. However, the absorbent article having the stretchy sheet 40 is not limited thereto, and examples thereof may include a pull-on disposable diaper for infants, a shorts-type napkin, a tape-type disposable diaper, an absorbent pad for light incontinence, a sanitary napkin, and like.

In the tape-type diaper 2, a pair of fastening tape portions 70 extend from the back portion toward two lateral outer sides. The wearer grasps the fastening tape portions 70 and pulls them up to the stomach side along the waist of the wearer, and then fastens the fastening tape portions 70 to the front portion of the tape-type diaper 2. Thus, it is preferable that the fastening tape portions 70 are configured with the stretchy sheets 40. According to this configuration, the wearer can easily pull up the fastening tape portions 70. In this case, the lateral direction of the stretchy sheet 40 (the direction along which the elastic members 3 extend) is aligned with the lateral direction (width direction) of the tape-type diaper 2, and the vertical direction of the stretchy sheet 40 (the direction perpendicular to the elastic members 3) is aligned with the vertical direction of the tape-type diaper 2 (the longitudinal direction of the absorbent core 11).

It is preferable that the spaced welded portion rows Ra are provided at two lateral ends of the fastening tape portions 70 (elastic members 3). According to this configuration, even if the fastening tape portions 70 are strongly pulled while the tape-type diaper 2 is putting on, it is possible to suppress the case where the elastic member 3 comes out of the spaced welded portion pair 51a. Meanwhile, it is preferable that the normal welded portion row Rb is provided in the lateral central portion of each of the fastening tapes 70. According to this configuration, the flexibility of the fastening tapes 70 is ensured.

Further, although not shown, the elastic members may be arranged in a portion other than the fastening tapes 70, such as in the lateral central portion of the back portion, and the elastic member may be sandwiched by the spaced welded portion pair 51a. In this case, as in FIG. 13 of the first embodiment, the spaced welded portion pairs 51a located in the fastening tapes 70 (the lateral end portions of the tape-type diaper 2) may have a longer lateral length than the spaced welded portion pairs 51a located at the lateral central portion of the back portion. According to this configuration, in the fastening tapes 70 that are strongly pulled, it is possible to more effectively suppress the case where the elastic member 3 comes out of the spaced welded portion pair 51a.

Further, the stretchy sheet 40 is not limited to being applied to the waist portion 20, 30 of the underpants-shaped diaper 1 or the fastening tapes 70 of the tape-type diaper 2, but may also be applied to other parts of the absorbent article, such as leg elastic portions and the like. Further, the stretchy sheet 40 may be applied to a product (e.g., a cleaning material, a mask, etc.) other than the absorbent article.

Embodiments of the present invention have been described above, however, embodiments of the present disclosure described above are simply to facilitate understanding of the present disclosure and are not in any way to be construed as limiting the present disclosure_{∘} The present disclosure may variously be changed or altered without departing from its essential features and encompass equivalents thereof.

**[Reference Signs List]**

| | |
|---|---|
| 1 | diaper (underpants-shaped absorbent article), |
| 2 | tape-type diaper, 3 elastic member, |
| 10 | absorbent main body, 11 absorbent core, 12 top sheet, |
| 13 | back sheet, 14 exterior sheet, 15 leg elastic member, |
| 16 | leak-proof wall elastic member, |
| 20 | front waist portion (waist portion), |
| 21 | skin-side sheet (sheet), 22 non-skin-side sheet (sheet), |
| 23 | waist elastic member (elastic member, first elastic member), |
| 23A | elastic fiber, |
| 30 | back waist portion (waist portion), |
| 30E | extension portion, |
| 31 | skin-side sheet (sheet), 32 non-skin-side sheet (sheet), |
| 33 | waist elastic member (elastic member), |
| 40 | stretchy sheet, |
| SS | side joining portion, |
| 50 | welded portion, |
| 501 | first welded portion, 502 second welded portion, |
| S1 | first space, |
| 51 | welded portion pair, |
| 51a | spaced welded portion pair, |
| 51a1 | first spaced welded portion pair (first welded portion pair), |
| 51a2 | second spaced welded portion pair (second welded portion pair), |
| 51b | normal welded portion pair, |
| 52 | non-welded portion, |
| 60, 61 | compressed portion, |
| 70 | fastening tape |

## Claims

1. A stretchy sheet (40) having a vertical direction and a lateral direction intersecting each other, the stretchy sheet (40) comprising:
a pair of sheets;
a plurality of welded portions (50) joining the pair of sheets; and
a plurality of elastic members (23) capable of stretching and contracting in the lateral direction,
the plurality of elastic members (23) being arranged with a space in the vertical direction and between the pair of sheets that are joined by the plurality of welded portions (50),
the plurality of welded portions including a plurality of welded portion pairs, the plurality of welded portion pairs including a welded portion pair (51a) located on two sides of an elastic member (23) in the vertical direction, the elastic member (23) being included in the plurality of elastic members (23),
the elastic member (23), in a state of contracting in the lateral direction, being sandwiched between the welded portion pair (51a), thereby restricting a position of the elastic member (23) in the lateral direction with respect to the pair of sheets,
the plurality of welded portion pairs including a first welded portion pair (51a1) and a second welded portion pair (51a2) that is located adjacent to the first welded portion pair (51a1) on one side in the lateral direction,
both the first welded portion pair (51a1) and the second welded portion pair (51a2) sandwiching a first elastic member (23), the first elastic member being included in the plurality of elastic members (23),
the first welded portion pair (51a1) including a first welded portion (501) and a second welded portion (502) that are located with a first space (Lb) therebetween in the lateral direction, on a same side in the vertical direction with respect to the first elastic member (23),
in a state where the stretchy sheet (40) is stretched in the lateral direction, a first length (D1) being a lateral length from a lateral end on the one side of the first welded portion pair (51a1) to a lateral end on another side of the second welded portion pair (51a2), the first length (D1) being longer than a lateral length of the first space (Lb).

2. The stretchy sheet according to claim 1, wherein
in the state where the stretchy sheet (40) is stretched in the lateral direction,
the lateral length (Lb) of the first space (S1) is
equal to or less than a lateral length (La) of the first welded portion (501), and
equal to or less than a lateral length (La) of the second welded portion (502).

3. The stretchy sheet according to claim 1 or 2, wherein
in the state where the stretchy sheet (40) is stretched in the lateral direction,
the first length (D1) is longer than a lateral length (La) of the first welded portion pair (51a1).

4. The stretchy sheet according to claim 1 or 2, wherein
the second welded portion pair (51a2) including a third welded portion (503) and a fourth welded portion (504) that are located with a second space (S2) therebetween in the lateral direction, on the same side in the vertical direction with respect to the first elastic member, and
in the state where the stretchy sheet (40) is stretched in the lateral direction, the first length (D1) is longer than a lateral length (Lb) of the second space (S2).

5. The stretchy sheet according to claim 1 or 2, wherein
the first welded portion pair (51a1) includes, on the same side in the vertical direction with respect to the first elastic member (23),
the first welded portion (501) and the second welded portion (502), and
one or more fifth welded portions (505) arranged to be spaced apart therefrom in the lateral direction, and
in the state where the stretchy sheet (40) is stretched in the lateral direction,
the first length (D1) is longer than a lateral length (Lb) of a space between the one fifth welded portion (505) and the first welded portion (501) or another fifth welded portion (505), the first welded portion (501) and the other fifth welded portion (505) being adjacent to the one fifth welded portion (505) in the lateral direction, the other fifth welded portion (505) being included in the fifth welded portions (505).

6. The stretchy sheet according to claim 5, wherein
in the state where the stretchy sheet (40) is stretched in the lateral direction,
the lateral length (Lb) of the space between the one fifth welded portion and the first welded portion or the other fifth welded portion adjacent to the one fifth welded portion in the lateral direction is equal to or less than each of lateral lengths of the first welded portion, the second welded portion, and the fifth welded portion.

7. The stretchy sheet according to claim 1 or 2, wherein
the first welded portion pair (51a) includes a sixth welded portion (506) and a seventh welded portion (507) that are located with a third space (S3) therebetween in the lateral direction, on a side opposite to the same side in the vertical direction with respect to the first elastic member (23), and
in the state where the stretchy sheet (40) is stretched in the lateral direction, the first length (D1) is longer than a lateral length of the third space (S3).

8. The stretchy sheet according to claim 7, wherein
in the state where the stretchy sheet (40) is stretched in the lateral direction,
the first space (S1) and the third space (S3) have a region in which the first space (S1) and the third space (S3) overlap in the lateral direction, and
a lateral center of the first space (S1) is shifted in the lateral direction from a lateral center of the third space (S3).

9. The stretchy sheet according to claim 8, further comprising:
a plurality of eighth welded portions (508) arranged to be spaced apart from the first welded portion (501) in the vertical direction, and
a plurality of ninth welded portions (509) arranged to be spaced apart from the second welded portion (502) in the vertical direction, wherein
an eighth welded portion (508) of the eighth welded portions is shifted in the lateral direction from the first welded portion (501) or another eighth welded portion of the eighth welded portions, the first welded portion and the other eighth welded portion being adjacent to the eighth welded portion in the vertical direction,
a ninth welded portion (509) of the ninth welded portions is shifted in the lateral direction from the second welded portion (502) or another ninth welded portion of the ninth welded portions, the second welded portion and the other ninth welded portion being adjacent to the ninth welded portion in the vertical direction, and
in the state where the stretchy sheet (40) is stretched in the lateral direction,
the first length (D1) is longer than a lateral length (Lb) of a space between the eighth welded portion and the ninth welded portion, the eighth welded portion and the ninth welded portion being adjacent in the lateral direction.

10. The stretchy sheet according to claim 1 or 2, wherein
each of the pair of sheets has a plurality of compressed portions (60, 61) in which the each of the pair of sheets is compressed in a thickness direction thereof, and
when the stretchy sheet (40) in a laterally stretched state is viewed from above,
at least a part of the plurality of compressed portions (60, 61) included in one of the pair of sheets has a region that is not overlapped with the compressed portions included in another sheet of the pair of sheets.

11. The stretchy sheet according to claim 1 or 2, wherein
at least one of the pair of sheets includes a plurality of compressed portions (60, 61) in which the at least one of the pair of sheets is compressed in a thickness direction thereof, and
when the stretchy sheet (40) in a laterally stretched state is viewed from above,
the first space (S1) has a region that is not overlapped with the compressed portions.

12. The stretchy sheet according to claim 1 or 2, wherein
at least one of the pair of sheets has a plurality of compressed portions (60, 61) in which the at least one of the pair of sheets is compressed in a thickness direction thereof, and
in the state where the stretchy sheet (40) is stretched in the lateral direction,
the lateral length (Lb) of the first space (S1) is equal to or less than a maximum value of a lateral interval between the compressed portions adjacent in the lateral direction.

13. The stretchy sheet according to claim 1 or 2, wherein
when twice a length of a thickness of at least one of the pair of sheets is defined as a second length, and
in the state where the stretchy sheet is stretched in the lateral direction, the lateral length of the first space is equal to or more than the second length.

14. The stretchy sheet according to claim 1 or 2, wherein
the first elastic member (23) in a twisted state is arranged between the pair sheets.

15. The stretchy sheet according to claim 1 or 2, wherein the first elastic member
is a string-like elastic member in which a plurality of elastic fibers (23A) are assembled, and
has a first outer diameter portion (23B) having an outer diameter that is a first outer diameter (d23B), and a second outer diameter portion (23C) having an outer diameter (d23C) smaller than the first outer diameter.

16. The stretchy sheet according to claim 1 or 2, wherein
the plurality of welded portion pairs include a third welded portion pair (51a3) that is located adjacent to the first welded portion pair (51a1) on the other side in the lateral direction, the third welded portion pair sandwiching the first elastic member, and
in the state where the stretchy sheet (40) is stretched in the lateral direction, a lateral length (D1) from a lateral end on the other side of the first welded portion pair to a lateral end on the one side of the third welded portion pair is longer than the lateral length (Lb) of the first space (S1).

17. An absorbent article (1, 2) including the stretchy sheet according to claim 1 or 2, the absorbent article having a vertical direction and a lateral direction intersecting each other, wherein
the vertical direction of the stretchy sheet (40) is aligned with the vertical direction of the absorbent article,
the lateral direction of the stretchy sheet (40) is aligned with the lateral direction of the absorbent article,
the first elastic member (23) includes a non-continuous portion in which the first elastic member (23) is cut to be non-continuous in a lateral central portion of the absorbent article, and continuous portions located on outer sides in the lateral direction with respect to the non-continuous portion, and
the first welded portion pair (51a1) sandwiches an end portion on a central side in the lateral direction of each of the continuous portions.

18. An absorbent article (1, 2) including the stretchy sheet according to claim 1 or 2, the absorbent article having a vertical direction and a lateral direction intersecting each other, wherein
the vertical direction of the stretchy sheet (40) is aligned with the vertical direction of the absorbent article,
the lateral direction of the stretchy sheet (40) is aligned with the lateral direction of the absorbent article,
the stretchy sheet has a fourth welded portion pair that sandwiches the first elastic member,
the fourth welded portion pair has the plurality of welded portions arranged with a space in the lateral direction, on the same side in the vertical direction with respect to the first elastic member,
the first welded portion pair is located in each lateral end portion of the absorbent article,
the fourth welded portion pair is located in a lateral central portion of the absorbent article, and
a lateral length (Lc) of the first welded portion pair is longer than a lateral length (Ld) of the fourth welded portion pair.

19. An underpants-shaped absorbent article (1, 2) including the stretchy sheet according to claim 1 or 2, the underpants-shaped absorbent article having a vertical direction and a lateral direction intersecting each other, the underpants-shaped absorbent article, comprising:
an absorbent main body (10): and
a pair of waist portions (20, 30),
the stretchy sheet (40) being provided to at least one of the pair of waist portions,
the vertical direction of the stretchy sheet being aligned with the vertical direction of the underpants-shaped absorbent article,
the lateral direction of the stretchy sheet being aligned with the lateral direction of the underpants-shaped absorbent article,
the pair of waist portions (20, 30) being joined, at lateral side portions thereof, by a pair of side joining portions, and
in the state where the underpants-shaped absorbent article is stretched in the lateral direction,
at least a part of the first welded portion pair (51a1) being located in a region that is located on an inner side in the lateral direction, by the first length, from a lateral inner end of each of the side joining portions.

## Patentansprüche

1. Dehnbare Lage (40), die eine vertikale Richtung und eine laterale Richtung aufweist, die einander schneiden, wobei die dehnbare Lage (40) Folgendes umfasst:
ein Paar von Lagen;
eine Vielzahl von verschweißten Teilen (50), die das Paar von Lagen verbinden; und
eine Vielzahl von elastischen Elementen (23), die sich in der lateralen Richtung dehnen und zusammenziehen können,
wobei die Vielzahl von elastischen Elementen (23) mit einem Abstand in der vertikalen Richtung und zwischen dem Paar von Lagen, die durch die Vielzahl von verschweißten Teilen (50) verbunden sind, angeordnet sind,
die Vielzahl von verschweißten Teilen eine Vielzahl von Paaren von verschweißten Teilen einschließt, wobei die Vielzahl von Paaren von verschweißten Teilen ein Paar von verschweißten Teilen (51a) einschließt, das sich auf zwei Seiten eines elastischen Elements (23) in der vertikalen Richtung befindet, wobei das elastische Element (23) in der Vielzahl von elastischen Elementen (23) eingeschlossen ist,
das elastische Element (23) in einem in der lateralen Richtung zusammengezogenen Zustand zwischen dem Paar von verschweißten Teilen (51a) eingefügt ist, wodurch eine Position des elastischen Elements (23) in der lateralen Richtung in Bezug auf das Paar von Lagen eingeschränkt ist,
die Vielzahl von Paaren von verschweißten Teilen Folgendes einschließt: ein erstes Paar von verschweißten Teilen (51a1) und ein zweites Paar von verschweißten Teilen (51a2), das sich benachbart zu dem ersten Paar von verschweißten Teilen (51a1) auf einer Seite in der lateralen Richtung befindet,
sowohl das erste Paar von verschweißten Teilen (51a1) als auch das zweite Paar von verschweißten Teilen (51a2) ein erstes elastisches Element (23) einfügen, wobei das erste elastische Element (23) in der Vielzahl von elastischen Elementen (23) eingeschlossen ist,
das erste Paar von verschweißten Teilen (51a1) einen ersten verschweißten Teil (501) und einen zweiten verschweißten Teil (502), die sich mit einem ersten Abstand (Lb) dazwischen in der lateralen Richtung befinden, auf einer gleichen Seite in der vertikalen Richtung in Bezug auf das erste elastische Element (23) einschließt,
in einem Zustand, in dem die dehnbare Lage (40) in der lateralen Richtung gedehnt ist, eine erste Länge (D1) eine laterale Länge von einem lateralen Ende auf der einen Seite des ersten Paars von verschweißten Teilen (51a1) zu einem lateralen Ende auf einer anderen Seite des zweiten Paars von verschweißten Teilen (51a2) ist, wobei die erste Länge (D1) länger ist als eine laterale Länge des ersten Abstands (Lb).

2. Dehnbare Lage nach Anspruch 1, wobei
in dem Zustand, in dem die dehnbare Lage (40) in der lateralen Richtung gedehnt ist,
die laterale Länge (Lb) des ersten Abstands (S1)
gleich oder kleiner als eine laterale Länge (La) des ersten verschweißten Teils (501) ist und
gleich oder kleiner als eine laterale Länge (La) des zweiten verschweißten Teils (502) ist.

3. Dehnbare Lage nach Anspruch 1 oder 2, wobei
in dem Zustand, in dem die dehnbare Lage (40) in der lateralen Richtung gedehnt ist,
die erste Länge (D1) länger ist als eine laterale Länge (La) des ersten Paars von verschweißten Teilen (51a1) ist.

4. Dehnbare Lage nach Anspruch 1 oder 2, wobei
das zweite Paar von verschweißten Teilen (51a2) einen dritten verschweißten Teil (503) und einen vierten verschweißten Teil (504), die sich mit einem zweiten Abstand (S2) dazwischen in der lateralen Richtung befinden, auf der gleichen Seite in der vertikalen Richtung in Bezug auf das erste elastische Element einschließt, und
in dem Zustand, in dem die dehnbare Lage (40) in der lateralen Richtung gedehnt ist, die erste Länge (D1) länger ist als eine laterale Länge (Lb) des zweiten Abstands (S2).

5. Dehnbare Lage nach Anspruch 1 oder 2, wobei
das erste Paar von verschweißten Teilen (51a1) auf der gleichen Seite in der vertikalen Richtung in Bezug auf das erste elastische Element (23) Folgendes einschließt:
den ersten verschweißten Teil (501) und den zweiten verschweißten Teil (502), und
ein oder mehrere fünfte verschweißte Teile (505), die angeordnet sind, um davon in der lateralen Richtung beabstandet zu sein, und
in dem Zustand, in dem die dehnbare Lage (40) in der lateralen Richtung gedehnt ist,
die erste Länge (D1) länger ist als eine laterale Länge (Lb) eines Abstands zwischen dem einen fünften verschweißten Teil (505) und dem ersten verschweißten Teil (501) oder einem anderen fünften verschweißten Teil (505), wobei der erste verschweißte Teil (501) und der andere fünfte verschweißte Teil (505) benachbart zu dem einen fünften verschweißten Teil (505) in der lateralen Richtung vorliegen, wobei der andere fünfte verschweißte Teil (505) in den fünften verschweißten Teilen (505) eingeschlossen ist.

6. Dehnbare Lage nach Anspruch 5, wobei
in dem Zustand, in dem die dehnbare Lage (40) in der lateralen Richtung gedehnt ist,
die laterale Länge (Lb) des Abstands zwischen dem einen fünften verschweißten Teil und dem ersten verschweißten Teil oder dem anderen fünften verschweißten Teil, die benachbart zu dem einen fünften verschweißten Teil in der lateralen Richtung vorliegen, gleich oder kleiner ist als jede der lateralen Längen des ersten verschweißten Teils, des zweiten verschweißten Teils und des fünften verschweißten Teils.

7. Dehnbare Lage nach Anspruch 1 oder 2, wobei
das erste Paar von verschweißten Teilen (51a) einen sechsten verschweißten Teil (506) und einen siebten verschweißten Teil (507), die sich mit einem dritten Abstand (S3) dazwischen in der lateralen Richtung befinden, auf einer Seite gegenüber der gleichen Seite in der vertikalen Richtung in Bezug auf das erste elastische Element (23) einschließt, und
in dem Zustand, in dem die dehnbare Lage (40) in der lateralen Richtung gedehnt ist, die erste Länge (D1) länger ist als eine laterale Länge des dritten Abstands (S3).

8. Dehnbare Lage nach Anspruch 7, wobei
in dem Zustand, in dem die dehnbare Lage (40) in der lateralen Richtung gedehnt ist,
der erste Abstand (S1) und der dritte Abstand (S3) einen Bereich aufweisen, in dem der erste Abstand (S1) und der dritte Abstand (S3) in der lateralen Richtung überlappen, und
eine laterale Mitte des ersten Abstands (S1) in der lateralen Richtung von einer lateralen Mitte des dritten Abstands (S3) verschoben ist.

9. Dehnbare Lage nach Anspruch 8, die weiter Folgendes umfasst:
eine Vielzahl von achten verschweißten Teilen (508), die angeordnet sind, um von dem ersten verschweißten Teil (501) in der vertikalen Richtung beabstandet zu sein, und
eine Vielzahl von neunten verschweißten Teilen (509), die angeordnet sind, um von dem zweiten verschweißten Teil (502) in der vertikalen Richtung beabstandet zu sein, wobei
ein achter verschweißter Teil (508) der achten verschweißten Teile in der lateralen Richtung von dem ersten verschweißten Teil (501) oder einem anderen achten verschweißten Teil der achten verschweißten Teile verschoben ist, wobei der erste verschweißte Teil und der andere achte verschweißte Teil benachbart zu dem achten verschweißten Teil in der vertikalen Richtung vorliegen,
ein neunter verschweißter Teil (509) der neunten verschweißten Teile in der lateralen Richtung von dem zweiten verschweißten Teil (502) oder einem anderen neunten verschweißten Teil der neunten verschweißten Teile verschoben ist, wobei der zweite verschweißte Teil und der andere neunte verschweißte Teil benachbart zu dem neunten verschweißten Teil in der vertikalen Richtung vorliegen, und
in dem Zustand, in dem die dehnbare Lage (40) in der lateralen Richtung gedehnt ist,
die erste Länge (D1) länger ist als eine laterale Länge (Lb) eines Abstands zwischen dem achten verschweißten Teil und dem neunten verschweißten Teil, wobei der achte verschweißte Teil und der neunte verschweißte Teil in der lateralen Richtung benachbart vorliegen.

10. Dehnbare Lage nach Anspruch 1 oder 2, wobei
jede des Paars von Lagen eine Vielzahl von komprimierten Teilen (60, 61) aufweist, wobei die jede des Paars von Lagen in einer Dickenrichtung davon komprimiert ist, und,
wenn die dehnbare Lage (40) in einem lateral gedehnten Zustand von oben betrachtet wird,
zumindest ein Teil der Vielzahl von komprimierten Teilen (60, 61), die in einer des Paars von Lagen eingeschlossen sind, einen Bereich aufweist, der nicht mit den komprimierten Teilen überlappt, die in einer anderen Lage des Paars von Lagen eingeschlossen sind.

11. Dehnbare Lage nach Anspruch 1 oder 2, wobei
zumindest eine des Paars von Lagen eine Vielzahl von komprimierten Teilen (60, 61) aufweist, wobei die mindestens eine des Paars von Lagen in einer Dickenrichtung davon komprimiert ist, und,
wenn die dehnbare Lage (40) in einem lateral gedehnten Zustand von oben betrachtet wird,
der erste Abstand (S1) einen Bereich aufweist, der nicht mit den komprimierten Teilen überlappt.

12. Dehnbare Lage nach Anspruch 1 oder 2, wobei
zumindest eine des Paars von Lagen eine Vielzahl von komprimierten Teilen (60, 61) aufweist, wobei die mindestens eine des Paars von Lagen in einer Dickenrichtung davon komprimiert ist, und
in dem Zustand, in dem die dehnbare Lage (40) in der lateralen Richtung gedehnt ist,
die laterale Länge (Lb) des ersten Abstands (S1) gleich oder kleiner ist als ein maximaler Wert einer lateralen Entfernung zwischen den komprimierten Teilen, benachbart in der lateralen Richtung.

13. Dehnbare Lage nach Anspruch 1 oder 2, wobei,
wenn eine doppelte Länge einer Dicke von mindestens einer des Paars von Lagen als eine zweite Länge definiert ist und
in dem Zustand, in dem die dehnbare Lage in der lateralen Richtung gedehnt ist, die laterale Länge des ersten Abstands gleich oder größer ist als die zweite Länge.

14. Dehnbare Lage nach Anspruch 1 oder 2, wobei
das erste elastische Element (23) in einem verdrehten Zustand zwischen dem Paar von Lagen angeordnet ist.

15. Dehnbare Lage nach Anspruch 1 oder 2, wobei
das erste elastische Element
ein fadenähnliches elastisches Element ist, in dem eine Vielzahl von elastischen Fasern (23A) zusammengefügt sind, und
Folgendes aufweist: einen ersten Außendurchmesserteil (23B), der einen Außendurchmesser aufweist, der ein erster Außendurchmesser (d23B) ist, und einen zweiten Außendurchmesserteil (23C), der einen Außendurchmesser (d23C) aufweist, der kleiner ist als der erste Außendurchmesser.

16. Dehnbare Lage nach Anspruch 1 oder 2, wobei
die Vielzahl von Paaren von verschweißten Teilen ein drittes Paar von verschweißten Teilen (51a3) einschließt, das sich benachbart zu dem ersten Paar von verschweißten Teilen (51a1) auf der anderen Seite in der lateralen Richtung befindet, wobei das dritte Paar von verschweißten Teilen das erste elastische Element einfügt, und
in dem Zustand, in dem die dehnbare Lage (40) in der lateralen Richtung gedehnt ist, eine laterale Länge (D1) von einem lateralen Ende auf der anderen Seite des ersten Paars von verschweißten Teilen zu einem lateralen Ende auf der einen Seite des dritten Paars von verschweißten Teilen länger ist als die laterale Länge (Lb) des ersten Abstands (S1).

17. Absorbierender Artikel (1, 2), der die dehnbare Lage nach Anspruch 1 oder 2 einschließt, wobei der absorbierende Artikel eine vertikale Richtung und eine laterale Richtung aufweist, die einander schneiden, wobei
die vertikale Richtung der dehnbaren Lage (40) mit der vertikalen Richtung des absorbierenden Artikels ausgerichtet ist,
die laterale Richtung der dehnbaren Lage (40) mit der lateralen Richtung des absorbierenden Artikels ausgerichtet ist,
das erste elastische Element (23) Folgendes einschließt: einen nicht-fortlaufenden Teil, in dem das erste elastische Element (23) geschnitten ist, um in einem lateralen Mittelteil des absorbierenden Artikels nicht fortlaufend zu sein, und fortlaufende Teile, die sich an Außenseiten in der lateralen Richtung in Bezug auf den nicht-fortlaufenden Teil befinden, und
das erste Paar von verschweißten Teilen (51a1) einen Endteil auf einer mittleren Seite in der lateralen Richtung von jedem der fortlaufenden Teile einfügt.

18. Absorbierender Artikel (1, 2), der die dehnbare Lage nach Anspruch 1 oder 2 einschließt, wobei der absorbierende Artikel eine vertikale Richtung und eine laterale Richtung aufweist, die einander schneiden, wobei
die vertikale Richtung der dehnbaren Lage (40) mit der vertikalen Richtung des absorbierenden Artikels ausgerichtet ist,
die laterale Richtung der dehnbaren Lage (40) mit der lateralen Richtung des absorbierenden Artikels ausgerichtet ist,
die dehnbare Lage ein viertes Paar von verschweißten Teilen aufweist, das das erste elastische Element einfügt,
das vierte Paar von verschweißten Teilen die Vielzahl von verschweißten Teilen, die mit einem Abstand in der lateralen Richtung angeordnet sind, auf der gleichen Seite in der vertikalen Richtung in Bezug auf das erste elastische Element aufweist,
sich das erste Paar von verschweißten Teilen in jedem lateralen Endteil des absorbierenden Artikels befindet,
sich das vierte Paar von verschweißten Teilen in einem lateralen mittleren Teil des absorbierenden Artikels befindet und
eine laterale Länge (Lc) des ersten Paars von verschweißten Teilen länger ist als eine laterale Länge (Ld) des vierten Paars von verschweißten Teilen.

19. Unterhosen-förmiger absorbierender Artikel (1, 2), der die dehnbare Lage nach Anspruch 1 oder 2 einschließt, wobei der Unterhosen-förmige absorbierende Artikel eine vertikale Richtung und eine laterale Richtung aufweist, die einander schneiden, wobei der Unterhosen-förmige absorbierende Artikel Folgendes umfasst:
einen absorbierenden Hauptkörper (10); und
ein Paar von Taillenteilen (20, 30),
wobei die dehnbare Lage (40) in mindestens einem des Paars von Taillenteilen bereitgestellt ist,
die vertikale Richtung der dehnbaren Lage mit der vertikalen Richtung des Unterhosen-förmigen absorbierenden Artikels ausgerichtet ist,
die laterale Richtung der dehnbaren Lage mit der lateralen Richtung des Unterhosen-förmigen absorbierenden Artikels ausgerichtet ist,
das Paar von Taillenteilen (20, 30) an lateralen Seitenteilen davon durch ein Paar von Seitenverbindungsteilen verbunden ist und
in dem Zustand, in dem der Unterhosen-förmige absorbierende Artikel in der lateralen Richtung gedehnt ist,
sich zumindest ein Teil des ersten Paars von verschweißten Teilen (51a1) in einem Bereich befindet, der sich auf einer Innenseite in der lateralen Richtung um die erste Länge von einem lateralen inneren Ende von jedem der Seitenverbindungsteile befindet.

## Revendications

1. Feuille extensible (40) ayant une direction verticale et une direction latérale qui se croisent l'une par rapport à l'autre, la feuille extensible (40) comportant :
une paire de feuilles ;
une pluralité de parties soudées (50) permettant d'assembler la paire de feuilles ; et
une pluralité d'éléments élastiques (23) en mesure de s'étirer et de se contracter dans la direction latérale,
les éléments de la pluralité d'éléments élastiques (23) étant agencés avec un espace dans la direction verticale et entre la paire de feuilles qui sont assemblées par la pluralité de parties soudées (50),
la pluralité de parties soudées comprenant une pluralité de paires de parties soudées, la pluralité de paires de parties soudées comprenant une paire de parties soudées (51a) se trouvant sur deux côtés d'un élément élastique (23) dans la direction verticale, l'élément élastique (23) étant inclus dans la pluralité d'éléments élastiques (23),
l'élément élastique (23), dans un état de contraction dans la direction latérale, étant pris en sandwich entre la paire de parties soudées (51a), pour de ce fait limiter une position de l'élément élastique (23) dans la direction latérale par rapport à la paire de feuilles,
la pluralité de paires de parties soudées comprenant une première paire de parties soudées (51a1) et une deuxième paire de parties soudées (51a2) qui est située de manière adjacente par rapport à la première paire de parties soudées (51a1) sur un côté dans la direction latérale,
à la fois, la première paire de parties soudées (51a1) et la deuxième paire de parties soudées (51a2) prenant en sandwich un premier élément élastique (23), le premier élément élastique étant inclus dans la pluralité d'éléments élastiques (23),
la première paire de parties soudées (51a1) comprenant une première partie soudée (501) et une deuxième partie soudée (502) qui sont situées avec un premier espace (Lb) entre elles dans la direction latérale, sur un même côté dans la direction verticale par rapport au premier élément élastique (23),
dans un état dans lequel la feuille extensible (40) est étirée dans la direction latérale, une première longueur (D1) étant une longueur latérale allant d'une extrémité latérale sur ledit un côté de la première paire de parties soudées (51a1) jusqu'à une extrémité latérale sur un autre côté de la deuxième paire de parties soudées (51a2), la première longueur (D1) étant plus longue qu'une longueur latérale du premier espace (Lb).

2. Feuille extensible selon la revendication 1, dans laquelle
dans l'état dans lequel la feuille extensible (40) est étirée dans la direction latérale,
la longueur latérale (Lb) du premier espace (S1) est
égale ou inférieure à une longueur latérale (La) de la première partie soudée (501), et
égale ou inférieure à une longueur latérale (La) de la deuxième partie soudée (502).

3. Feuille extensible selon la revendication 1 ou la revendication 2, dans laquelle
dans l'état dans lequel la feuille extensible (40) est étirée dans la direction latérale,
la première longueur (D1) est plus longue qu'une longueur latérale (La) de la première paire de parties soudées (51a1).

4. Feuille extensible selon la revendication 1 ou la revendication 2, dans laquelle
la deuxième paire de parties soudées (51a2) comprend une troisième partie soudée (503) et une quatrième partie soudée (504) qui sont situées avec un deuxième espace (S2) entre elles dans la direction latérale, sur le même côté dans la direction verticale par rapport au premier élément élastique, et
dans l'état dans lequel la feuille extensible (40) est étirée dans la direction latérale, la première longueur (D1) est plus longue qu'une longueur latérale (Lb) du deuxième espace (S2).

5. Feuille extensible selon la revendication 1 ou la revendication 2, dans laquelle
la première paire de parties soudées (51a1) comprend, sur le même côté dans la direction verticale par rapport au premier élément élastique (23),
la première partie soudée (501) et la deuxième partie soudée (502), et
une ou plusieurs cinquièmes parties soudées (505) agencées de manière à être espacées par rapport à celles-ci dans la direction latérale, et
dans l'état dans lequel la feuille extensible (40) est étirée dans la direction latérale,
la première longueur (D1) est plus longue qu'une longueur latérale (Lb) d'un espace entre ladite une cinquième partie soudée (505) et la première partie soudée (501) ou une autre cinquième partie soudée (505), la première partie soudée (501) et ladite autre cinquième partie soudée (505) étant adjacentes par rapport à la cinquième partie soudée (505) dans la direction latérale, l'autre cinquième partie soudée (505) étant incluse dans les cinquièmes parties soudées (505).

6. Feuille extensible selon la revendication 5, dans laquelle
dans l'état dans lequel la feuille extensible (40) est étirée dans la direction latérale,
la longueur latérale (Lb) de l'espace entre ladite une cinquième partie soudée et la première partie soudée ou ladite autre cinquième partie soudée adjacente par rapport à ladite une cinquième partie soudée dans la direction latérale est égale ou inférieure à chacune des longueurs latérales de la première partie soudée, de la deuxième partie soudée et de la cinquième partie soudée.

7. Feuille extensible selon la revendication 1 ou la revendication 2, dans laquelle
la première paire de parties soudées (51a) comprend une sixième partie soudée (506) et une septième partie soudée (507) qui sont situées avec un troisième espace (S3) entre elles dans la direction latérale, sur un côté opposé au même côté dans la direction verticale par rapport au premier élément élastique (23), et
dans l'état dans lequel la feuille extensible (40) est étirée dans la direction latérale, la première longueur (D1) est plus longue qu'une longueur latérale du troisième espace (S3).

8. Feuille extensible selon la revendication 7, dans laquelle
dans l'état dans lequel la feuille extensible (40) est étirée dans la direction latérale,
le premier espace (S1) et le troisième espace (S3) ont une région dans laquelle le premier espace (S1) et le troisième espace (S3) se chevauchent dans la direction latérale, et
un centre latéral du premier espace (S1) est décalé dans la direction latérale par rapport à un centre latéral du troisième espace (S3).

9. Feuille extensible selon la revendication 8, comportant par ailleurs :
une pluralité de huitièmes parties soudées (508) agencées de manière à être espacées par rapport à la première partie soudée (501) dans la direction verticale, et
une pluralité de neuvièmes parties soudées (509) agencées de manière à être espacées par rapport à la deuxième partie soudée (502) dans la direction verticale, dans laquelle
une huitième partie soudée (508) des huitièmes parties soudées est décalée dans la direction latérale par rapport à la première partie soudée (501) ou une autre huitième partie soudée des huitièmes parties soudées, la première partie soudée et l'autre huitième partie soudée étant adjacentes par rapport à la huitième partie soudée dans la direction verticale,
une neuvième partie soudée (509) des neuvièmes parties soudées est décalée dans la direction latérale par rapport à la deuxième partie soudée (502) ou d'une autre neuvième partie soudée des neuvièmes parties soudées, la deuxième partie soudée et l'autre neuvième partie soudée étant adjacentes par rapport à la neuvième partie soudée dans la direction verticale, et
dans l'état dans lequel la feuille extensible (40) est étirée dans la direction latérale,
la première longueur (D1) est plus longue qu'une longueur latérale (Lb) d'un espace entre la huitième partie soudée et la neuvième partie soudée, la huitième partie soudée et la neuvième partie soudée étant adjacentes dans la direction latérale.

10. Feuille extensible selon la revendication 1 ou la revendication 2, dans laquelle
chaque feuille de la paire de feuilles a une pluralité de parties comprimées (60, 61) dans lesquelles ladite chaque feuille de la paire de feuilles est comprimée dans une direction allant dans le sens de son épaisseur, et
quand la feuille extensible (40) dans un état étiré latéralement est vue de dessus,
au moins une partie de la pluralité de parties comprimées (60, 61) incluses dans une feuille de la paire de feuilles a une région qui n'est pas chevauchée par les parties comprimées incluses dans une autre feuille de la paire de feuilles.

11. Feuille extensible selon la revendication 1 ou la revendication 2, dans laquelle
au moins une feuille de la paire de feuilles comprend une pluralité de parties comprimées (60, 61) dans lesquelles ladite au moins une feuille de la paire de feuilles est comprimée dans une direction allant dans le sens de son épaisseur, et
quand la feuille extensible (40) dans un état étiré latéralement est vue de dessus,
le premier espace (S1) a une région qui n'est pas chevauchée par les parties comprimées.

12. Feuille extensible selon la revendication 1 ou la revendication 2, dans laquelle
au moins une feuille de la paire de feuilles a une pluralité de parties comprimées (60, 61) dans lesquelles ladite au moins une feuille de la paire de feuilles est comprimée dans une direction allant dans le sens de son épaisseur, et
dans l'état dans lequel la feuille extensible (40) est étirée dans la direction latérale,
la longueur latérale (Lb) du premier espace (S1) est égale ou inférieure à une valeur maximale d'un intervalle latéral entre les parties comprimées adjacentes dans la direction latérale.

13. Feuille extensible selon la revendication 1 ou la revendication 2, dans laquelle
lorsque deux fois une longueur d'une épaisseur d'au moins une feuille de la paire de feuilles est définie comme étant une deuxième longueur, et
dans l'état dans lequel la feuille extensible est étirée dans la direction latérale, la longueur latérale du premier espace est égale ou supérieure à la deuxième longueur.

14. Feuille extensible selon la revendication 1 ou la revendication 2, dans laquelle
le premier élément élastique (23) dans un état torsadé est agencé entre la paire de feuilles.

15. Feuille extensible selon la revendication 1 ou la revendication 2, dans laquelle le premier élément élastique
est un élément élastique de type ficelle dans lequel une pluralité de fibres élastiques (23A) sont assemblées, et
a une première partie formant diamètre extérieur (23B) ayant un diamètre extérieur qui est un premier diamètre extérieur (d23B), et une deuxième partie formant diamètre extérieur (23C) ayant un diamètre extérieur (d23C) inférieur au premier diamètre extérieur.

16. Feuille extensible selon la revendication 1 ou la revendication 2, dans laquelle
la pluralité de paires de parties soudées comprend une troisième paire de parties soudées (51a3) qui est située de manière adjacente par rapport à la première paire de parties soudées (51a1) sur l'autre côté dans la direction latérale, la troisième paire de parties soudées prenant en sandwich le premier élément élastique, et
dans l'état dans lequel la feuille extensible (40) est étirée dans la direction latérale, une longueur latérale (D1) allant d'une extrémité latérale sur l'autre côté de la première paire de parties soudées jusqu'à une extrémité latérale sur ledit un côté de la troisième paire de parties soudées est plus longue que la longueur latérale (Lb) du premier espace (S1).

17. Article absorbant (1, 2) comprenant la feuille extensible selon la revendication 1 ou la revendication 2, l'article absorbant ayant une direction verticale et une direction latérale qui se croisent l'une par rapport à l'autre, dans lequel
la direction verticale de la feuille extensible (40) est alignée sur la direction verticale de l'article absorbant,
la direction latérale de la feuille extensible (40) est alignée sur la direction latérale de l'article absorbant,
le premier élément élastique (23) comprend une partie non continue dans laquelle le premier élément élastique (23) est coupé de manière à être non continu dans une partie centrale latérale de l'article absorbant, et des parties continues situées sur des côtés extérieurs dans la direction latérale par rapport à la partie non continue, et
la première paire de parties soudées (51a1) prend en sandwich une partie d'extrémité sur un côté central dans la direction latérale de chacune des parties continues.

18. Article absorbant (1, 2) comprenant la feuille extensible selon la revendication 1 ou la revendication 2, l'article absorbant ayant une direction verticale et une direction latérale qui se croisent l'une par rapport à l'autre, dans lequel
la direction verticale de la feuille extensible (40) est alignée sur la direction verticale de l'article absorbant,
la direction latérale de la feuille extensible (40) est alignée sur la direction latérale de l'article absorbant,
la feuille extensible a une quatrième paire de parties soudées qui prend en sandwich le premier élément élastique,
la quatrième paire de parties soudées a la pluralité de parties soudées agencées avec un espace dans la direction latérale, sur le même côté dans la direction verticale par rapport au premier élément élastique,
la première paire de parties soudées est située dans chaque partie d'extrémité latérale de l'article absorbant,
la quatrième paire de parties soudées est située dans une partie centrale latérale de l'article absorbant, et
une longueur latérale (Lc) de la première paire de parties soudées est plus longue qu'une longueur latérale (Ld) de la quatrième paire de parties soudées.

19. Article absorbant en forme de culotte (1, 2) comprenant la feuille extensible selon la revendication 1 ou la revendication 2, l'article absorbant en forme de culotte ayant une direction verticale et une direction latérale qui se croisent l'une par rapport à l'autre, l'article absorbant en forme de culotte comportant :
un corps principal absorbant (10) : et
une paire de parties au niveau de la taille (20, 30),
la feuille extensible (40) étant fournie à au moins une partie de la paire de parties au niveau de la taille,
la direction verticale de la feuille extensible étant alignée sur la direction verticale de l'article absorbant en forme de culotte,
la direction latérale de la feuille extensible étant alignée sur la direction latérale de l'article absorbant en forme de culotte,
les parties de la paire de parties au niveau de la taille (20, 30) sont assemblées, au niveau des parties côté latéral de celles-ci, par une paire de parties d'assemblage latérales, et
dans l'état dans lequel l'article absorbant en forme de culotte est étiré dans la direction latérale,
au moins une partie de la première paire de parties soudées (51a1) est située dans une région qui est située sur un côté intérieur dans la direction latérale, par la première longueur, à partir d'une extrémité intérieure latérale de chacune des parties d'assemblage latérales.
